# EUROPEAN PATENT SPECIFICATION

(11) **EP 3 045 181 B1**
(45) Date of publication and mention of the grant of the patent: **14.11.2018**
(21) Application number: 15151594.7
(22) Date of filing: 19.01.2015
(51) Int. Cl.: C07K 14/005, A61K 39/12

(54) **A novel vaccine against the middle east respiratory syndrome coronavirus (MERS-CoV)**
Neuartiger Impfstoff gegen das Nahost-Atemwegssyndrom-Coronavirus (MERS-CoV)
Nouveau vaccin contre le coronavirus du syndrome respiratoire du moyen orient (MERS-CoV)

(43) Date of publication of application: 20.07.2016
(73) Proprietor: Ludwig-Maximilians-Universität München, 80539 München (DE); Philipps-Universität Marburg, 35032 Marburg (DE)
(72) Inventor: Sutter, Gerd, 80803 München (DE); Fux, Robert, 85368 Wang (DE); Volz, Asisa, 80799 München (DE); Song, Fei, 80805 München (DE); Becker, Stephan, 35037 Marburg (DE); Eickmann, Markus, 35096 Weimar (DE)
(74) Representative: Vossius & Partner Patentanwälte Rechtsanwälte mbB

(56) References cited:
- WO-A1-2015/081155
- WO-A2-2014/045254
- ARWEN ALTENBURG ET AL: "Modified Vaccinia Virus Ankara (MVA) as Production Platform for Vaccines against Influenza and Other Viral Respiratory Diseases", VIRUSES, vol. 6, no. 7, 17 July 2014 (2014-07-17), pages 2735-2761, XP055200206, DOI: 10.3390/v6072735
- F. SONG ET AL: "Middle East Respiratory Syndrome Coronavirus Spike Protein Delivered by Modified Vaccinia Virus Ankara Efficiently Induces Virus-Neutralizing Antibodies", JOURNAL OF VIROLOGY, vol. 87, no. 21, 1 November 2013 (2013-11-01), pages 11950-11954, XP055157123, ISSN: 0022-538X, DOI: 10.1128/JVI.01672-13
- RACHEL L. GRAHAM ET AL: "A decade after SARS: strategies for controlling emerging coronaviruses", NATURE REVIEWS MICROBIOLOGY, vol. 11, no. 12, 11 November 2013 (2013-11-11), pages 836-848, XP055200225, ISSN: 1740-1526, DOI: 10.1038/nrmicro3143
- S. VAN BOHEEMEN ET AL: "Genomic Characterization of a Newly Discovered Coronavirus Associated with Acute Respiratory Distress Syndrome in Humans", MBIO, vol. 3, no. 6, 20 November 2012 (2012-11-20), pages e00473-12, XP055128733, ISSN: 2150-7511, DOI: 10.1128/mBio.00473-12 & "Human betacoronavirus 2c EMC/2012, complete genome, GeneBank accession number JX869059", EMBL, 20 November 2012 (2012-11-20), XP002727344, [retrieved on 2012-09-28]
- RUTH MCBRIDE ET AL: "The Coronavirus Nucleocapsid Is a Multifunctional Protein", VIRUSES, vol. 6, no. 8, 7 August 2014 (2014-08-07), pages 2991-3018, XP055200197, DOI: 10.3390/v6082991
- ZHAO JINCUN ET AL: "Airway Memory CD4(+) T Cells Mediate Protective Immunity against Emerging Respiratory Coronaviruses.", IMMUNITY 21 JUN 2016, vol. 44, no. 6, 21 June 2016 (2016-06-21), pages 1379-1391, ISSN: 1097-4180
- ZHAO JINCUN ET AL: "Rapid generation of a mouse model for Middle East respiratory syndrome.", PROCEEDINGS OF THE NATIONAL ACADEMY OF SCIENCES OF THE UNITED STATES OF AMERICA 01 APR 2014, vol. 111, no. 13, 1 April 2014 (2014-04-01), pages 4970-4975, ISSN: 1091-6490

## Description

The present disclosure relates to the Middle East Respiratory Syndrome Coronavirus (MERS-CoV) N nucleocapsid protein and/or an immunogenic fragment thereof, or a nucleic acid molecule encoding the MERS-CoV N nucleocapsid protein and/or the immunogenic fragment thereof, for use as a vaccine. The present invention further relates to an immunogenic composition comprising the MERS-CoV N nucleocapsid protein and/or an immunogenic fragment thereof, or a nucleic acid molecule encoding the MERS-CoV N nucleocapsid protein and/or the immunogenic fragment thereof. Furthermore, the present invention relates to a vector comprising a nucleic acid molecule encoding the MERS-CoV N nucleocapsid protein and/or an immunogenic fragment thereof, for use as a vaccine as well as a method of inducing a protective immune response against MERS-CoV.

Middle East Respiratory Syndrome (MERS) is a viral respiratory illness first reported in Saudi Arabia in 2012 accompanied by fever, cough, and shortness of breath. Most infections were geographically linked to the Middle East, i.e., Jordan, Saudi Arabia, Qatar, and the United Arab Emirates, but cases also occurred in the United Kingdom, Germany, France, and Italy. In the same year, the Middle East Respiratory Syndrome Coronavirus (MERS-CoV) emerged as the causative agent of the infection in humans. At present, human cases of MERS-CoV infections continue to be reported. The virus is thought to persist in animal reservoirs, in particular in dromedary camels, and to cause sporadic zoonotic infections followed by intra-familial or health-care-related transmissions. However, further details on the epidemiology of MERS-CoV infection remain unclear. As of 14 Nov 2014, the global tally of reports by local health authorities worldwide on cases of MERS-CoV was 941, including 375 deaths (case fatality rate >39 per cent).

Treatment of the disease is currently attempted via administration of interferon to reduce viral replication *in vitro,* or via inhibition of the viral protease. However, so far no medication has been proven to treat MERS-CoV, and treatment is mostly based upon the patient's medical condition.

An alternative approach for the treatment of such virally caused diseases is the development of vaccines to protect from an infection. In this regard, Song *et al*. 2013 reported the efficient induction of virus-neutralising antibodies against MERS-CoV based on the MERS-CoV spike (S) protein, a characteristic structural component of the virion membrane that forms large protruding spikes on the surface of the virus. Additionally, experimental vaccines also targeting the MERS-CoV S antigen and based on recombinant protein, nanoparticles or an adenovirus vector have been described recently (Ma et al. Vaccine 2014 32:2100-2108; Coleman et al. Vaccine 2014 32:3169-3174; Kim et al. Vaccine 2014 32: 5975-5982).

However, despite the fact that a lot of effort is currently being invested into methods of providing treatments, and in particular vaccines, for MERS-CoV, there is still a need to provide additional or improved approaches against this virus.

This need is addressed by the provision of the embodiments characterised in the claims.

Accordingly, the present invention relates to an immunogenic composition comprising the MERS-CoV N nucleocapsid protein or a nucleic acid molecule encoding the MERS-CoV N nucleocapsid protein, wherein the composition further comprises at least one additional immunogenic compound and wherein the additional immunogenic compound is the MERS-CoV S spike protein.

The term "Middle East Respiratory Syndrome Coronavirus" is also abbreviated herein as MERS-CoV and is a group IV ((+)ssRNA) virus of the genus *betacoronavirus* following the nomenclature of the Coronavirus Study group (de Groot 2013). This virus was first described as human coronavirus EMC in 2012 by Zaki *et al*. (2012), Bermingham *et al.* (2012), van Boheemen *et al*. (2012) as well as Müller *et al*. 2012. The complete genome of the human *betacoronavirus* 2c EMC/2012 has been deposited under the GenBank accession number JX869059.2. In addition, the World Health Organization (WHO) maintains a website (see the World Wide Web at who.int/csr/disease/ coronavirus_infections/en/) reporting on the newest developments and information regarding this virus.

The "MERS-CoV N nucleocapsid protein", also referred to herein as "MERS-CoV N protein", is a protein of 413 amino acids length that shares a high homology with the N protein of other coronaviruses. The primary role of CoV N protein is the packaging of the genomic viral genome into long, flexible, helical ribonucleoprotein (RNP) complexes, the so-called nucleocapsids or capsids. The nucleocapsid protects the genome and ensures its timely replication and reliable transmission. In addition, studies have indicated that the N proteins of coronavirus also participate in the replication and transcription of viral RNA and interfere with the cell cycle processes of the host cells. Accordingly, CoV N proteins localize to the cytoplasm as well as to the nucleolus of the virus. The N nucleocapsid protein has been described e.g. in Masters & Perlman "Coronaviridae", p. 825-858, Fields Virology, 6th Edition 2013, Knipe DM, Howley PM Eds, Lippincott Williams & Wilkins, Philadelphia or in McBride *et al.* 2014. The N protein of the human *betacoronavirus* 2c EMC/2012 is represented by the GenBank accession number AFS88943.1 and is also shown in SEQ ID NO:1. In accordance with the present invention, embodiments wherein the MERS-CoV N protein (or fragment thereof) is present or used in the context of the whole MERS-CoV, i.e. in combination with all other components that make up this virus, are excluded.

The term "immunogenic fragment", as used herein, refers to a fragment of the MERS-CoV N nucleocapsid protein that maintains the ability of the full length MERS-CoV N nucleocapsid protein to elicit an immunogenic reaction i.e. its ability to stimulate the immune system of a subject, such as e.g. a human subject, to recognize it as foreign, destroy it, and subsequently enable the immune system to protect the subject against the disease for which the vaccine has been developed.

It is well known in the art that immunogenic protein may be cleaved to yield fragments which retain or essentially retain the immunogenic activity of the full length protein. Such cleavage may include the removal of a given number of N- and/or C-terminal amino acids, or the removal of a number of internal (non-terminal) amino acids. Said number of amino acids to be removed from the termini and/or internal regions may for example be one, two, three, four, five, six, seven, eight, nine, ten, 15, 20, 25, 30, 40, 50 or more than 50. Preferably, the fragment has a length of at least 6 amino acids, more preferably at least 7 amino acids, such as at least 8 amino acids, at least 9 amino acids, at least 10 amino acids, at least 11 amino acids, at least 12 amino acids, at least 13 amino acids, at least 14 amino acids, and most preferably at least 15 amino acids.
Means and methods for determining whether the immunogenic activity is retained are well known in the art and include e.g. experimental means such as the systematic generation of deletion mutants and their assessment in assays for immunogenic activity. For example, an immunogenic reaction of the fragment to be tested can be analysed by standard and routine techniques, e.g. by administering (e.g. via i.v. injection) the fragment of interest to a mammal such as a mouse and analysing the response of immunogenic blood cells and/or factors (e.g. interleukins) after an appropriate time for an immune reaction to occur. This can for example be done by applying serum from the animals inoculated with the fragment to a peptide array comprising the complete antigen. These and other means and methods for determining whether the immunogenic activity is retained are known to the skilled person and have been described in the art.

The immunogenic activity of a fragment of the MERS-CoV N nucleocapsid protein is essentially retained if at least 60% of the immunogenic activity of the full length MERS-CoV N nucleocapsid protein is retained. Preferably, at least 75% or more preferably at least 80% of the immunogenic activity of the full length MERS-CoV N nucleocapsid protein is retained. More preferred is that at least 90% such as at least 95%, even more preferred at least 98% such as at least 99% of the immunogenic activity of the full length MERS-CoV N nucleocapsid protein is retained. Most preferred is that immunogenic activity is fully, i.e. to 100%, retained. Also described are fragments having an increased immunogenic activity compared to the full length MERS-CoV N nucleocapsid protein, i.e. more than 100% activity. Preferably, the method to determine whether these percentages of immunogenic activity are retained is by measuring the induction of MERS CoV neutralising antibody titers in serum after immunisation, as shown in the appended examples using mice. A direct comparison of the immunogenic activity of such a fragment can be achieved by carrying out the same experiment using a full length MERS-CoV N nucleocapsid protein as well as the fragment of interest. However, it will be appreciated that standard values for the full length MERS-CoV N nucleocapsid protein may generated in advance and stored for comparison, thereby rendering the parallel comparison unnecessary if the same experimental conditions are observed.

It is also envisaged that a mixture of the full length MERS-CoV N nucleocapsid protein with (a) fragment(s) thereof is employed.

The MERS-CoV N nucleocapsid protein or an immunogenic fragment thereof can be expressed recombinantly by methods known in the art, e.g. by expressing a vector encoding the MERS-CoV N nucleocapsid protein or the fragment thereof in a suitable host and purifying the expressed MERS-CoV N nucleocapsid protein or the fragment thereof.
A large number of suitable methods exist in the art to produce proteins or peptides in appropriate hosts. If the host is a unicellular organism such as a prokaryote, a mammalian or insect cell, the person skilled in the art can revert to a variety of culture conditions. Conveniently, the produced protein is harvested from the culture medium, lysates of the cultured organisms or from isolated (biological) membranes by established techniques. In the case of a multi-cellular organism, the host may be a cell which is part of or derived from a part of the organism, for example said host cell may be the harvestable part of a plant. A preferred method involves the recombinant production of proteins in hosts as indicated above. For example, nucleic acid sequences comprising a polynucleotide encoding the MERS-CoV N nucleocapsid protein or a fragment thereof can be synthesized *in vitro* by gene synthesis or PCR and inserted into an expression vector. Subsequently a suitable host may be transformed with the expression vector. Thereafter, the host is cultured to produce the desired polypeptide(s), which is/are isolated and purified. An alternative method for producing the MERS-CoV N nucleocapsid protein or a fragment thereof is *in vitro* translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant polypeptides upon the addition of cloning vectors, DNA fragments, or RNA sequences containing coding regions and appropriate promoter elements.
In addition to recombinant production, the protein or fragments of the invention may be produced synthetically, e.g. by direct peptide synthesis using solid-phase techniques (cf Stewart et al. (1969) Solid Phase Peptide Synthesis; Freeman Co, San Francisco; Merrifield, J. Am. Chem. Soc. 85 (1963), 2149-2154). Synthetic protein synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule. As indicated above, chemical synthesis, such as the solid phase procedure described by Houghton Proc. Natl. Acad. Sci. USA (82) (1985), 5131-5135, can be used. Furthermore, the protein or fragments of the protein of the invention may be produced semi-synthetically, for example by a combination of recombinant and synthetic production.
Protein isolation and purification can be achieved by any one of several known techniques; for example and without limitation, ion exchange chromatography, gel filtration chromatography and affinity chromatography, high pressure liquid chromatography (HPLC), reversed phase HPLC, and preparative disc gel electrophoresis. Protein isolation/purification techniques may require modification of the proteins of the present invention using conventional methods. For example, a histidine tag can be added to the protein to allow purification on a nickel column. Other modifications may cause higher or lower activity, permit higher levels of protein production, or simplify purification of the protein.

In accordance with the present invention, the term "nucleic acid molecule", also referred to as nucleic acid sequence herein, includes DNA, such as cDNA or genomic DNA, and RNA. It is understood that the term "RNA" as used herein comprises all forms of RNA including mRNA, tRNA and rRNA. Both, single-strand as well as double-strand nucleic acid molecules are encompassed by this term. Further included are nucleic acid mimicking molecules known in the art such as synthetic or semi-synthetic derivatives of DNA or RNA and mixed polymers, both sense and antisense strands. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey (2001) Chem Biol. 8, 1). LNA is an RNA derivative in which the ribose ring is constrained by a methylene linkage between the 2'-oxygen and the 4'-carbon. They may contain additional non-natural or derivatised nucleotide bases, as will be readily appreciated by those skilled in the art.

The nucleic acid molecule encoding the MERS-CoV N nucleocapsid protein and/or a fragment thereof can be combined, preferably in a vector, with additional elements, such as e.g. regulatory elements. Regulatory elements are required to ensure expression of the encoded protein after incorporation thereof in the host cell(s) of the organism to be vaccinated, to thereby synthesize the antigen. Regulatory elements are also required to ensure replication of the nucleic acid molecule in particular in those cases where the newly introduced nucleic acid molecule is present as an extra-chromosomal sequence or where regulation via endogenous regulatory elements is not ensured. Non-limiting examples of regulatory elements include an origin of replication, a promoter as well as terminating sequences. Additional regulatory elements may include translational enhancers and translation initiation codons, Shine-Dalgarno boxes or internal ribosomal entry sites or signal sequences capable of directing the expressed fusion protein to a specific cellular compartment. When the newly introduced nucleic acid molecule is carried by a poxvirus DNA genome, replication is also possible without the presence of an origin of replication.

The promoter initiates transcription of the nucleic acid molecule and can be used to control the level of gene expression, while the terminator sequence ends transcription. Preferably, the promoter should be highly active. It is further preferred that the promoter is a native and conserved promoter that is independent of inducing systems, such as e.g. IPTG or arabinose. Non-limiting examples of promoters include the natural vaccinia virus promoters P7.5 or P11, the synthetic vaccinia virus early-late promoters sP (Chakrabarti et al. Biotechniques 1997, 23:1094), PmH5 (Wyatt et al. Vaccine 1996 14:1451), sPII (Wyatt et al. AIDS Res Hum Retroviruses. 2004 Jun;20(6):645-53), the cytomegalovirus (CMV) promoter, SV40-promoter, RSV-promoter (Rous sarcoma virus), the CAG promoter, the gai10 promoter, human elongation factor 1a-promoter, CMV early enhancer or the SV40-enhancer.

Most preferably, the promoter is the synthetic vaccinia virus promoter PmH5 employed in the appended examples.

Non-limiting examples for regulatory elements ensuring transcription termination include the vaccina virus TTTTTNT early termination signal, the SV40-poly-A site or the tk-poly-A site or the SV40, lacZ polyadenylation signals, downstream of the polynucleotide.

Preferably, the vector is a virus, plasmid, cosmid or bacteriophage. The nucleic acid molecule of the present invention may be inserted into any one of several commercially available vectors. Suitable expression vectors which comprise the described regulatory elements are known in the art such as Okayama-Berg cDNA expression vector pcDV1 (Pharmacia), pRc/CMV, pcDNA1, pcDNA3, pSFV1 (In-Vitrogene). Most preferably, the vector is a virus as described in more detail herein below.

The nucleic acid sequences inserted in the vector can e.g. be synthesized by standard chemical synthesis methods, or isolated from natural sources or produced semi-synthetically, i.e. by combining chemical synthesis and isolation from natural sources. Ligation of the coding sequences to transcriptional regulatory elements and/or to other amino acid encoding sequences can be carried out using established methods, such as restriction digests, ligations and molecular cloning. Vector modification techniques belong to the standard techniques in the field of molecular biology and, thus, belong to the scientific knowledge of the skilled person.

The term "vaccine", as used herein, is defined in accordance with the pertinent art and relates to a composition that induces or enhances the protective immunity of an individual to a particular disease caused by a pathogen. Without wishing to be bound by theory, it is believed that a protective immunity arises from the generation of neutralizing antibodies, or from the activation of cytotoxic cells of the immune system, or both.

In order to induce or enhance a protective immunity, the vaccine comprises as an immunogenic antigen a part of the pathogen causing said disease or a nucleic acid molecule encoding this immunogenic antigen. In the present case, the immunogenic antigen is the MERS-CoV N nucleocapsid protein, or an immunogenic fragment thereof (or a mixture of the full length MERS-CoV N nucleocapsid protein with (a) fragment(s) thereof) and the pathogen is MERS-CoV, which causes the Middle East Respiratory Syndrome. Upon contact with the immunogenic antigen, the immune system of the individual is triggered to recognise the immunogenic antigen as foreign and to destroy it. The immune system subsequently remembers the contact with this immunogenic antigen, so that at a later contact with the disease-causing pathogen an easy and efficient recognition and destruction of the pathogen is ensured.

The vaccine may be for use in preventing, alleviating and/or treating Middle East Respiratory Syndrome. Preferably, the vaccine is for use in preventing Middle East Respiratory Syndrome.

The vaccine may be in any formulation for vaccines known in the art, such as for example mucosal vaccines, vaccines for intramuscular injection or vaccines for subcutaneous or intradermal injection as well as vaccines for inhalation, such as e.g. as aerosols. Such vaccine formulations are well known in the art. Accordingly, the vaccine may be in liquid, aerosolic, or solid form and may be, inter alia, in the form of (a) solution(s), (a) spray(s), (a) powder(s) or (a) tablet(s) or paste(s).

In accordance with the present invention, it was surprisingly found that the MERS-CoV N nucleocapsid protein is recognised by MERS-CoV-specific antibodies in macaque serum, obtained from an MERS-CoV infected cynomolgous macaque. These findings indicate a particular immunogenicity of the MERS-CoV N nucleocapsid.

The MERS-CoV genome contains 30119 nucleotides, and encodes four major structural proteins, the spike (S) protein, envelope (E) protein and membrane (M) protein, as well as the nucleocapsid (N) protein (van Boheemen *et al*., 2012). The spike (S) protein has previously been shown to be an important component of a candidate vaccine (Song *et al*., 2013) and the co-delivery of several membrane/envelope proteins (i.e. E or M proteins) was found to elicit higher levels of target virus specific immune responses or to elicit better protective capacity (Wyatt, 1996; Stittelaar 2000). However, the now identified role of the N protein comes as a surprise, as previous studies indicated that N proteins of coronaviruses participate in the replication and transcription of viral RNA and interfere with the cell cycle processes of the host cells. As such, the MERS-CoV N nucleocapsid protein is present only at the inside of the virus and inside of the infected cells and, accordingly, would not be expected to be able to trigger a protective immunogenic response of the infected host.

As is shown in the appended examples, novel recombinant viruses of Modified Vaccinia virus Ankara (MVA) stably containing gene sequences encoding the full-length MERS-CoV proteins S and N were constructed. The recombinant MVA viruses amplified to high titers in chicken embryo fibroblasts but failed to grow in human Hela and HaCat cells suggesting their suitability for vaccine development. Vero cells infected with MVA-MERS-SN co-produced an about 210-kDa MERS-CoV S glycoprotein and an about 45 kDa MERS-CoV N nucleocapsid protein. When comparing the detection of both recombinant antigens by serum antibodies raised in MERS-CoV infected macaques, the N protein was much more prominently recognized than the S protein showing a high level of antigenicity of the MERS-CoV N protein. More importantly, intramuscular vaccination of BALB/c mice with MVA-MERS-SN confirmed the particular immunogenicity of the recombinant N protein. Vaccination raised high levels of serum antibodies that reacted with the authentic N protein of MERS-CoV. Moreover, MVA-MERS-SN immunization elicited serum antibodies that neutralized a MERS-CoV *in vitro* infection more efficiently compared to antibodies induced by vaccination with a recombinant MVA vaccine delivering the S protein only (MVA-MERS-S). Since MVA-MERS-S and MVA-MERS-SN are based on the identical clonal isolate the improved immunogenicity of MVA-MERS-SN is directly related to the presence of MERS-CoV N in the composition.

The present disclosure further relates to an immunogenic composition comprising the MERS-CoV N nucleocapsid protein and/or an immunogenic fragment thereof, or a nucleic acid molecule encoding the MERS-CoV N nucleocapsid protein and/or the immunogenic fragment thereof.

The term "immunogenic composition", as used herein, relates to a composition capable of eliciting an immunogenic reaction in mammals, preferably in mouse, rat and/or human, most preferably in human. The person skilled in the art can determine immunogenic reactions by standard and routine techniques, as detailed herein above with regard to the determination of an immunogenic activity of proteins or fragments thereof.

In accordance with the present invention, the term "composition" relates to a composition which comprises at least one of the compounds of the invention. The composition of the present invention may, optionally and additionally, comprise further compounds such as e.g. a pharmaceutically acceptable carrier or further molecules capable of altering the characteristics of the compounds of the invention thereby, for example, stabilizing, delaying, modulating and/or activating their function. Non-limiting examples of such compounds are described further below. In accordance with the present invention, the composition does not comprise (or consist of) the whole MERS-CoV, i.e. the MERS-CoV N protein in combination with all other components that make up this virus.
Compositions according to the present invention can be formulated by well known conventional methods. The composition may be in solid, liquid or gaseous form and may be, inter alia, in the form of (a) powder(s), (a) tablet(s), (a) solution(s) or (an) aerosol(s).
These compositions can be administered to a subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The effective amount for a given situation will readily be determined by routine experimentation and is within the skills and judgement of the ordinary clinician or physician. For example, if the MERS-CoV N protein is the sole immunogenic compound, the effective amount administered per dose will be in the range of about 1 µg kg to 10 mg kg of patient body weight, although, as noted above, this will be subject to therapeutic discretion. More preferably, this dose is at least 0.01 mg kg, and most preferably for humans between about 0.01 and 1 mg kg. The particular amounts, as well as the corresponding adjustments in case more than one compound is to be administered, may be determined by conventional tests which are well known to the person skilled in the art. Administration may be once as a single dose or as repeat administrations, such as e.g. a primary immunisation and subsequent boost immunisations. The interval time and amount of boost immunisations required can be determined by the skilled person without further ado. Preferably, the administration comprises a primary immunisation and at least one boost immunisation. More preferably, the boost immunisation is repeated at least twice. Even more preferably, the boost immunisation is repeated weekly, monthly or yearly. The compositions of the invention may be administered orally, intradermally, subcutaneously, intramuscularly, intraperitoneally, topically (as by powders, ointments, drops or transdermal patch), bucally, or as an oral or nasal spray.

The term "comprising", as used throughout the present invention, denotes that further components can be included in addition to the specifically recited components, with the proviso that the sum of all these components does not represent or make up the whole MERS-CoV. However, this term also encompasses that the claimed subject-matter consists of exactly the recited components.

All other definitions and preferred embodiments provided herein above with regard to the MERS-CoV N nucleocapsid protein and/or an immunogenic fragment thereof for use as a vaccine apply *mutatis mutandis* also to this immunogenic composition of the invention.

Preferably, the immunogenic composition is a vaccine, as defined herein above. In other words, this embodiment of the present invention preferably relates to a composition comprising the MERS-CoV N nucleocapsid protein and/or an immunogenic fragment thereof, or a nucleic acid molecule encoding the MERS-CoV N nucleocapsid protein and/or the immunogenic fragment thereof, for use as a vaccine.

The immunogenic composition may further comprise at least one additional compound selected from the group consisting of immunogenic compounds, adjuvants, pharmaceutically acceptable carriers, stabilisers and/or preservatives.

The term "at least one", as used herein, encompasses also at least two, such as at least three, at least four or at least five or more, such as at least ten. It will be appreciated by the skilled person that this term further encompasses exactly one, exactly two, exactly three, exactly four, exactly five or exactly ten.

An immunogenic compound can be any compound, or mixtures of compounds, capable of eliciting an immunogenic reaction, as defined herein above. Preferably, the immunogenic compound is an immunogenic MERS-CoV protein, or a fragment thereof. As discussed herein above, immunogenic MERS-CoV proteins include the S protein, the E protein and M protein as well as the proteins 3, 4a, 4b and 5. These proteins are well known in the art and their amino acid and nucleic acid sequences have been recorded under the following database accession numbers: S protein (GenBank: AFS88936.1 for amino acid sequence and GenBank: JX869059.2 for nucleic acid sequence), the E protein (GenBank: AFS88941.1 for amino acid sequence and GenBank: JX869059.2 for nucleic acid sequence) and M protein GenBank: AFS88942.1 for amino acid sequence and GenBank: JX869059.2 for nucleic acid sequence) as well as the proteins 3 (GenBank: AFS88937.1 for amino acid sequence and GenBank: JX869059.2 for nucleic acid sequence), 4a (GenBank: AFS88938.1 for amino acid sequence and GenBank: JX869059.2 for nucleic acid sequence), 4b (GenBank: AFS88939.1 for amino acid sequence and GenBank: JX869059.2 for nucleic acid sequence) and 5 (GenBank: AFS88940.2 for amino acid sequence and GenBank: JX869059.2 for nucleic acid sequence). In addition, the amino acid sequences of these proteins are represented by SEQ ID NOs: 3 and 9 to 14.

It will be appreciated that the MERS-CoV N nucleocapsid protein and an immunogenic fragment thereof are not encompassed by this definition of additional compounds present in the composition of the invention. In other words, in those cases where the composition further comprises an additional compound and said additional compounds is an immunogenic compound, the immunogenic compound is not the MERS-CoV N nucleocapsid protein and is not an immunogenic fragment thereof.

The term "adjuvant", as used herein, relates to one or more compounds that enhance the recipient's immune response to a vaccine. Adjuvants are often added to promote an earlier, more potent response, and/or more persistent immune response to the vaccine, which often allows for a lower vaccine dosage. Non-limiting examples of adjuvants include e.g. aluminium hydroxide and aluminium phosphate, the organic compound Squalene but also compounds such as e.g. ligands of the Toll-like receptors, QS21, aluminium hydroxide derivates, oil immersions, Lipid A and it's derivates (e.g. monophosphoryl lipid A (MPL), CpG motives, poly I:C dsRNA, Muramyldipeptid (MDP), Freund's Complete Adjuvant (FCA, for non-human use only), Freund's incomplete Adjuvant (FIA, for non-human use only) or MF59C. Such adjuvants are well known in the art.

Preferably, the adjuvants are ligands of the Toll-like receptors (TLR), such as e.g. dsRNA for TLR3, unmethylated CpG oligodesoxynucleotides for TLR9 or flagellin for TLR5.

In those cases where the immunogenic composition of the present invention comprises the MERS-CoV N nucleocapsid protein and/or an immunogenic fragment thereof, the additional immunogenic compound and/or adjuvant can be admixed thereto by conventional means. For example, an adjuvant such as e.g. CpG oligodesoxynucleotides or poly I:C dsRNA, or a mixture of both, can be directly admixed with the MERS-CoV N nucleocapsid protein and/or an immunogenic fragment thereof, or with a vector expressing same. Such approaches are known in the art and have been described, e.g. in Berchtold *et al.* 2009.
In those cases where the immunogenic composition of the present invention comprises a nucleic acid molecule encoding the MERS-CoV N nucleocapsid protein and/or an immunogenic fragment thereof, the additional immunogenic compound or adjuvant can be admixed thereto by conventional means, either as described above, or as (a) separate nucleic acid molecule(s) encoding said additional immunogenic compound or adjuvant. Alternatively, the nucleic acid sequence comprising the nucleic acid molecule encoding the MERS-CoV N nucleocapsid protein and/or the immunogenic fragment thereof may additionally also comprise the nucleic acid molecule(s) encoding said additional immunogenic compound and/or adjuvant. For example, a dsRNA ligand can be encoded on the same vector as the MERS-CoV N antigen, such as e.g. described by Scallan *et al.* 2012. A further example has been described by Arimilli *et al.* 2008, who showed that intracellular expression of the TLR5 ligand flagellin by paramyxovirus simian virus 5 (SV5) acts as an adjuvant that enhances the activation of SV5-infected dendritic cells.

The term "pharmaceutically acceptable carrier" relates to non-toxic excipients, including e.g. solid, semisolid or liquid fillers, diluents, binding agents, lubricants, various types of wetting agents, encapsulating material or formulation auxiliaries of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc.

Stabilisers are employed to prevent alterations of the vaccine when exposed to e.g. heat, light, acidity or humidity. Non-limiting examples of often used stabilisers include monosodium glutamate (MSG) and 2-phenoxyethanol.

Preservatives are typically added to prevent serious adverse side effects such as infection with bacteria or viruses grown in the vaccine during production or storage. Non-limiting examples of preservatives include antibiotics, formaldehyde, phenoxyethanol or thiomersal, which are usually added to vials of vaccine that contain more than one dose to prevent contamination and growth of potentially harmful bacteria/viruses.

Compositions comprising such pharmaceutically acceptable carriers, stabilisers and/or preservatives can be formulated by well known conventional methods.

The present disclosure further relates to a vector comprising a nucleic acid molecule encoding the MERS-CoV N nucleocapsid protein and/or an immunogenic fragment thereof, for use as a vaccine.

Suitable vectors for nucleic acid expression have been described herein above. The vector has to be a vector suitable for expression of the nucleic acid molecule in the host cell(s), i.e. after application of the vector as the vaccine. To this end, the vector comprises a nucleic acid molecule encoding the MERS-CoV N nucleocapsid protein and/or an immunogenic fragment thereof, as also discussed herein above. Moreover, the vector comprises said nucleic acid molecule "in expressible form", i.e. in a form that enables the expression of the respective expression product encoded by said nucleic acid molecule. Expression of a nucleic acid molecule can be ensured, for example, by employing regulatory elements, as discussed herein above.

In accordance with the present invention, the vector comprising a nucleic acid molecule encoding the MERS-CoV N nucleocapsid protein and/or an immunogenic fragment thereof does not comprise the entire MERS-CoV genome, such that the vector does not comprise or represent a whole MERS-CoV.

In a preferred embodiment of the vector of the invention, the vector is a virus.

The term "virus" as used herein is well known in the art. Non-limiting examples of viruses include vaccinia viruses, avipoxviruses, adenoviruses, alphaviruses, rhabdoviruses, herpesviruses, influenza viruses, polio viruses, arboviruses, astroviruses, bacteriophages, enteroviruses, gastroenteritis viruses, hantaviruses, coxsackie viruses, hepatitis A viruses, hepatitis B viruses, hepatitis C viruses, norwalk viruses, chordopoxyiridae (i.e., 5 orthopoxviruses), NYVAC, ALVAC, ALVAC(2), fowlpox, reoviruses, rhinoviruses, rotaviruses, retroviruses, baculoviridae, caliciviridae, caulimoviridae, coronaviridae, filoviridae, flaviviridae, hepadnaviridae, nodaviridae, orthomyxoviridae, paramyxoviridae, papovaviridae, parvoviridae, phycodnaviridae, picornaviridae, and togaviridae, and modified viruses originating therefrom. Other viruses are known and described in the art. In accordance with the present invention, the virus is not MERS-CoV.

In a further preferred embodiment of the vector of the invention, the vector is selected from the group consisting of vaccinia virus, avipoxvirus, adenovirus, alphavirus, rhabdovirus, and herpesvirus.

Vaccinia virus (VACV or VV) is a large, complex, enveloped virus belonging to the orthopoxvirus genus in the poxvirus family. It has a linear, double-stranded DNA genome approximately 190 kbp in length, which encodes for ∼ 250 genes. Vaccinia virus is best known for its role as a vaccine that eradicated the smallpox disease, making it the first human disease to be successfully eradicated by science.

Non-limiting examples of vaccinia virus strains to be used as vectors include the modified vaccinia virus Ankara (MVA), as well as Lister/Elstree, Dryvax, New York City Board of Health, EM63, ACAM2000, LC16m8, NYVAC, Tiantan, Western Reserve, Copenhagen or Connaught Laboratories. All these vaccinia strains are well known and described in the art.

Avipoxviruses are members of the Poxviridae family, a family of viruses which cause the victim organism to have pox lesions as a symptom. Members of the avipoxvirus genus infect specifically birds and are unable to complete their replication cycle in non-avian species.

Non-limiting examples of avipoxviruses include the canarypox virus, as well as fowlpox virus, turkeypox virus, falconpox virus and others. Avipoxviruses are well known and described in the art. Preferably, the avipoxvirus is the canarypox virus.

Adenoviruses are medium-sized (90-100 nm), non-enveloped icosahedral viruses composed of a nucleocapsid and a double-stranded linear DNA genome (group I (dsDNA)). Adenoviruses are for example responsible for respiratory diseases, conjunctivitis as well as gastroenteritis. Adenoviruses are commonly used as vectors to administer targeted therapy, for example in the form of recombinant DNA or protein.

Non-limiting examples of adenoviruses include human adenoviruses (hAd) such as hAd5 or hAd35, chimpanzee adenoviruses (ChAd) such as ChAd3, ChAd63, and rhesus monkey adenoviruses. All these adenoviruses are well known and described in the art.

Alphaviruses belong to the group IV Togaviridae family of viruses, which have a positive sense, single-stranded RNA genome. They are able to infect various vertebrates such as humans, rodents, fish, birds, and larger mammals such as horses as well as invertebrates. Alphavirus particles are enveloped and have a diameter of about 70 nm.

Non-limiting examples of alphaviruses include the Semliki Forest virus, as well as Ross River virus, Sindbis virus and Venezuelan equine encephalitis virus, all of which have been used to develop viral vectors for gene delivery. Alphaviruses or alphavirus replicons to be used as vectors are well known and described in the art. Preferably, the alphavirus is the Semliki Forest virus.

Rhabdoviruses are viruses belonging to the family Rhabdoviridae and infect a broad range of hosts throughout the animal and plant kingdoms. Animal rhabdoviruses infect insects, fish, and mammals, including humans. Rhabdoviruses carry their genetic material in the form of negative-sense single-stranded RNA and typically carry genes for five proteins: large protein (L), glycoprotein (G), nucleoprotein (N), phosphoprotein (P), and matrix protein (M).

Several genera of rhabdoviruses are known, including viruses of the genera Ephemerovirus, Lyssavirus, Novirhabdovirus, and Vesiculovirus. E.g. lyssaviruses and vesiculoviruses are developed as vectors and well known and described in the art. Preferably, the rhabdovirus is the vesicular stomatitis virus strain Indiana, also often referred to as the vesicular stomatitis virus.

Herpesviruses belong to a large family of DNA viruses composed of relatively large double-stranded, linear DNA genomes encoding 100-200 genes encased within an icosahedral protein cage (i.e. the capsid) which is wrapped in a protein layer called the tegument containing both viral proteins and viral mRNAs and an envelope, which is a lipid bilayer membrane.

Several subfamilies and genera of herpesviruses are presently known, of which herpes simplex virus-1 (HSV-1), herpes simplex virus-2 (HSV-2), Varicella zoster virus (VZV), Epstein-Barr virus (EBV), Cytomegalovirus (CMV) and Kaposi's sarcoma-associated herpesvirus (KSHV) are presently known as herpesviruses causing disease in humans. These herpesviruses are well known and described in the art. Preferably, the herpesvirus is Cytomegalovirus (CMV).

In a more preferred embodiment of the vector of the invention, the vector is a vaccinia virus.

In an even more preferred embodiment, the vaccinia virus is a modified vaccinia virus Ankara (MVA).

Modified vaccinia virus Ankara (MVA) is a highly attenuated strain of vaccinia virus that was created by passaging vaccinia virus several hundred times in chicken embryo fibroblasts (CEF), thereby achieving growth selection. MVA shows a characteristic replication defect in mammalian cells (Mayr and Munz 1964; Meyer *et al.* 1991; Sutter and Moss 1992) and, thus, serves as one of the most advanced recombinant poxvirus vectors in preclinical research and human clinical trials for developing new vaccines against infectious disease and cancer (Gilbert 2013; Kremer *et al.* 2012; Volz and Sutter 2013).

Means and methods for introducing the nucleic acid molecule in accordance with the invention into a MVA are well known in the art and have been described e.g. in Drexler *et al.* 2000; Staib *et al.* 2004; or Kremer *et al.* 2012 as well as in the appended examples.

Preferably, the nucleic acid molecule encoding the MERS-CoV N nucleocapsid protein and/or immunogenic fragment thereof is introduced into the MVA genome at an intergenic site and under transcriptional control of the vaccinia virus early/late promoter PmH5. More preferably, the nucleic acid molecule is introduced into the MVA genome at intergenic site 069R-070L (genes MVA-069 and MVA-070 as specified in GenBank: AY603355.1), as shown in the appended examples.

The vector may further comprise one or more nucleic acid molecules encoding a compound selected from the group consisting of additional immunogenic compounds and adjuvants.

It will be appreciated that in this regard, the immunogenic compounds and adjuvants have to be encoded by a nucleic acid molecule in order to be comprised by the vector of the invention. The definitions provided herein above with regard to additional immunogenic compounds and adjuvants, as well as nucleic acid molecule encoding same, apply *mutatis mutandis.*

The MERS-CoV N nucleocapsid protein may comprise or consist of the amino acid sequence of SEQ ID NO:1 or a functional variant thereof.

The term "functional variant", as used herein, relates to a MERS-CoV N nucleocapsid protein differing in its amino acid sequence from the sequence of SEQ ID NO:1 but retaining or essentially retaining the immunogenic activity of the MERS-CoV N nucleocapsid protein consisting of SEQ ID NO:1. Means and methods for measuring the immunogenic activity of a functional variant of the MERS-CoV N nucleocapsid protein have been detailed herein above with regard to fragments of the MERS-CoV N nucleocapsid protein. The same definitions and preferred embodiments, including the percentage of immunogenic activity that is considered to fall under the terms "retained or essentially retained immunogenic activity" apply *mutatis mutandis* to functional variants of the MERS-CoV N nucleocapsid proteins having additional amino acids. Described is that the amino acid sequence of the functional variant has at least 85% sequence identity to the amino acid sequence of SEQ ID NO:1 or at least 90%. According to the invention, sequence identity to the amino acid sequence of SEQ ID NO:1 is at least 95%. Described is also 97%, at least 98% and at least 99% sequence identity to the amino acid sequence of SEQ ID NO:1.

In accordance with the present invention, the term "% sequence identity" describes the number of matches ("hits") of identical amino acids of two or more aligned amino acid sequences as compared to the number of amino acids making up the overall length of the amino acid sequences (or the overall compared part thereof). In other terms, using an alignment, for two or more sequences the percentage of amino acids that are the same or similar may be determined, when the sequences are compared and aligned for maximum correspondence over a window of comparison, or over a designated region as measured using a sequence comparison algorithm as known in the art, or when manually aligned and visually inspected. Those having skill in the art know how to determine percent sequence identity between sequences using, for example, algorithms such as those based on the NCBI BLAST algorithm, CLUSTAL W computer program or FASTA, as known in the art. The NCBI BLAST algorithm is preferably employed in accordance with this invention. The BLASTP program for amino acid sequences uses as default a word length (W) of 3, and an expectation (E) of 10. The BLOSUM62 scoring matrix uses alignments (B) of 50, expectation (E) of 10, M=5, N=4, and a comparison of both strands.
Preferably, the cited "% sequence identity with SEQ ID NO:1" is over the entire length of SEQ ID NO:1.

Where the MERS-CoV N nucleocapsid protein comprises (rather than consists of) the sequence of SEQ ID NO:1 or a functional variant thereof, additional amino acids extend over the specific sequence either at the N-terminal end or the C-terminal end or both. Preferably, no more than 500 additional amino acids are present at the N- terminal end and no more than 500 additional amino acids are present at the C-terminal end. More preferably no more than 400, such as no more than 300, more preferably no more than 200, such as no more than 100, no more than 90, no more than 80, no more than 70, no more than 60, no more than 50, no more than 40, no more than 30, no more than 20 and even more preferably no more than 10 additional amino acids are independently present at either one or both of the N- or C-terminal end. Most preferably, no more than 5 additional amino acids are independently present at either one or both of the N- or C-terminal end. In those cases where such additional amino acids are comprised in the MERS-CoV N nucleocapsid protein, it is preferred that the MERS-CoV N nucleocapsid protein having these additional amino acids retains or essentially retains the immunogenic activity of the MERS-CoV N nucleocapsid protein consisting of the sequence of SEQ ID NO:1, as defined herein above.

Preferably, the MERS-CoV N nucleocapsid protein in accordance with the present invention is a protein comprising or consisting of the sequence of SEQ ID NO:1. Even more preferably, the MERS-CoV N nucleocapsid protein is a protein consisting of the sequence of SEQ ID NO:1.

The MERS-CoV N nucleocapsid protein consisting of the amino acid sequence of SEQ ID NO:1 corresponds to the MERS-CoV N nucleocapsid protein employed in the examples described herein below. Preferably, said MERS-CoV N nucleocapsid protein is encoded by the nucleic acid sequence shown in SEQ ID NO:2. However, it will be appreciated that further nucleic acid sequences are also suitable to encode the amino acid sequence of SEQ ID NO:1. This is due to the degeneracy of the genetic code and the fact that certain host cells may show an improved translation of the desired MERS-CoV N nucleocapsid protein if the encoding nucleic acid sequence is codon optimised for the translational machinery of these particular host cells. All these nucleic acid sequences are also encompassed herein for use in generating the MERS-CoV N nucleocapsid protein in accordance with the present invention.

The immunogenic fragment of the MERS-CoV N nucleocapsid protein may have a length of at least 6 amino acids, as described herein above. The immunogenic fragment of the MERS-CoV N nucleocapsid protein may have a length of between 12 and 18 amino acids.

In accordance with the invention, the additional immunogenic compound is the MERS-CoV S spike protein and/or an immunogenic fragment thereof as defined in the claims.

The MERS-CoV S spike protein is a characteristic structural component of the MERS-CoV virion membrane that forms large protruding spikes on the surface of the virus. Its S1 domain mediates binding to dipeptidyl-peptidase 4, which serves as the host cell receptor of MERS-CoV. The MERS-CoV S spike protein is considered a key component of vaccines against coronavirus infection and has recently been shown to be an important component of a candidate vaccine (Song *et al*., 2013).

The definition of the term "immunogenic fragment" as provided herein above with regard to the MERS-CoV N protein applies *mutatis mutandis* to the immunogenic fragment of the MERS-CoV S spike protein. Preferred length of such fragments, methods of determining whether an immunogenic activity is retained and percentages of retained immunogenic activity provided herein above with regard to the MERS-CoV N protein also apply to this immunogenic fragment of the MERS-CoV S spike protein.

As shown in the appended examples, intramuscular vaccination of BALB/c mice using a recombinant MVA virus stably containing gene sequences encoding the full-length MERS-CoV proteins S and N elicited serum antibodies that neutralized a MERS-CoV *in vitro* infection more efficiently compared to antibodies induced by vaccination with a recombinant MVA vaccine delivering the S protein only (MVA-MERS-S). At present, it is not known how the N protein - which is believed to be present only at the inside of the virus and inside of the infected cells - triggers an immunogenic response of the infected host. Nonetheless, the present findings clearly show that the additional presence of the MERS-CoV N gene sequences in the composition surprisingly provides an improved immunogenicity as compared to the use of MERS-CoV S spike protein alone. Possible mechanisms underlying this function may be the preferred recognition of MVA-MERS-S+N infected and antigen producing cells by professional antigen presenting cells such as macrophages or dendritic cells, the induction of cytokine responses and/or the improved attraction of immune cells to the site of MVA-MERS-S+N inoculation.

In a more preferred embodiment of the immunogenic composition or the vector of the invention the MERS-CoV S spike protein comprises or consists of the amino acid sequence of SEQ ID NO:3 or a functional variant thereof, said functional variant being as defined in the claims.

All of the definitions and preferred embodiments provided herein above apply *mutatis mutandis* to this embodiment.

The MERS-CoV S spike protein consisting of the amino acid sequence of SEQ ID NO:3 corresponds to the MERS-CoV S spike protein employed in the examples described herein below. Preferably, said MERS-CoV S spike protein is encoded by the nucleic acid sequence shown in SEQ ID NO:4. However, it will be appreciated that further nucleic acid sequences are also suitable to encode the amino acid sequence of SEQ ID NO:3 and all these nucleic acid sequences are also encompassed herein for use in generating the MERS-CoV S spike protein.

The immunogenic fragment of the MERS-CoV S spike protein may be the receptor binding domain (RBD) or parts thereof. Most preferably, the immunogenic fragment of the MERS-CoV S spike protein is the receptor binding domain (RBD). This receptor binding domain (RBD) of the MERS-CoV S spike protein is represented by amino acid residues 358 to 588 of the amino acid sequence of SEQ ID NO:3.

Described is also a method of inducing a protective immune response against MERS-CoV, the method comprising administering the immunogenic composition or the vector of the invention to a subject in need thereof.

In accordance with the present invention, the term "inducing a protective immune response" refers to an activation of the host's immune system to be able to prevent or to respond to a MERS-CoV infection. By administering the immunogenic composition or the vector of the invention to a subject, a vaccination against MERS-CoV is achieved as detailed herein above.

As also detailed above, the skilled person knows that the effective amount that needs to be administered to an individual in order to induce a protective immune response will, *inter alia*, depend on the individual to be treated. Such effective amounts can be determined by the skilled person without further ado.

Unless otherwise defined, all technical and scientific terms used herein have the same meaning as commonly understood by one of ordinary skill in the art to which this invention belongs. In case of conflict, the patent specification, including definitions, will prevail.

All the sequences accessible through the Database Accession numbers cited herein are within the scope of the present invention irrespective of whether the entry of the respective Accession No. is completely identical to the sequence displayed by the corresponding SEQ ID NO due to potential future updates in the database. Thus, this is to account for future corrections and modifications in the entries of GenBank, which might occur due to the continuing progress of science.

As regards the embodiments characterised in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims. To give a non-limiting example, the combination of claims 8, 7 and 6 is clearly and unambiguously envisaged in view of the claim structure. The same applies for example to the combination of claims 8 and 4, etc..

The figures show:

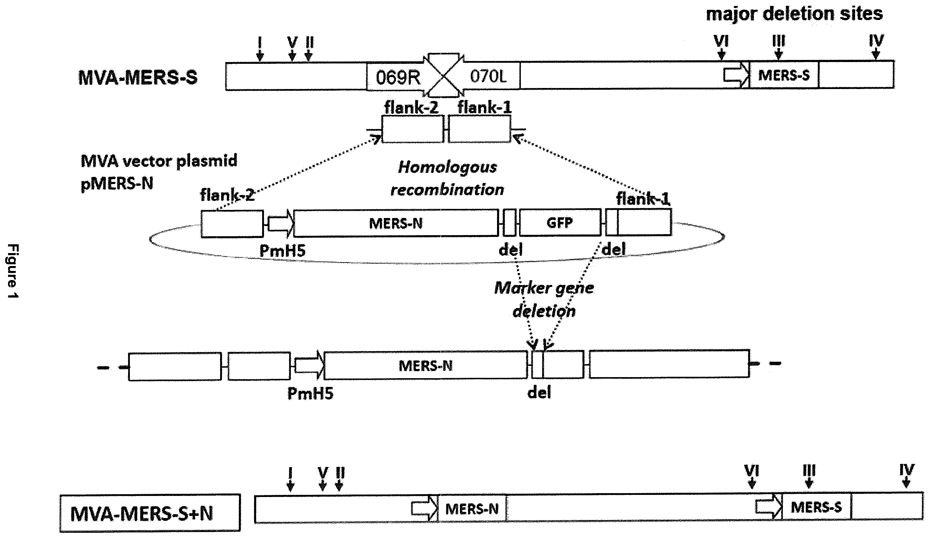

**Figure 1****: Construction of the recombinant virus MVA-MERS-S+N.** A schematic representation of the genome of MVA-MERS-S is shown on the top. The locations of the major natural deletion sites I to VI are indicated by arrow heads. MVA-MERS-S carries an expression cassette for the MERS-CoV S protein (MERS-S) inserted at the site of the natural deletion site III of the MVA genome. Synthetic gene sequences encoding the full-length MERS-CoV N protein (MERS-N) were placed under transcriptional control of the synthetic vaccinia virus promoter PmH5 and flanked by MVA DNA sequences (flank1, flank2) to precisely target the insertion of the heterologous sequences to the intergenic site 069R/070L of the MVA-MERS-S genome. In addition, the MVA vector plasmid pMERS-N contained gene sequences encoding the green fluorescent protein (GFP) as marker and the short repetitive sequence del derived from the flank1 sequence. MVA-MERS-S+N was generated by homologous recombination following transfection of vector plasmid DNA (pMERS-N) and co-infection with the clonal recombinant virus MVA-MVA-MERS-S. Double recombinant viruses were isolated in plaque purification passages on chicken embryo fibroblasts through screening for transient coproduction of GFP. The marker gene was removed by intragenomic homologous recombination between the del sequences to yield the final vector virus MVA-MERS-S+N.

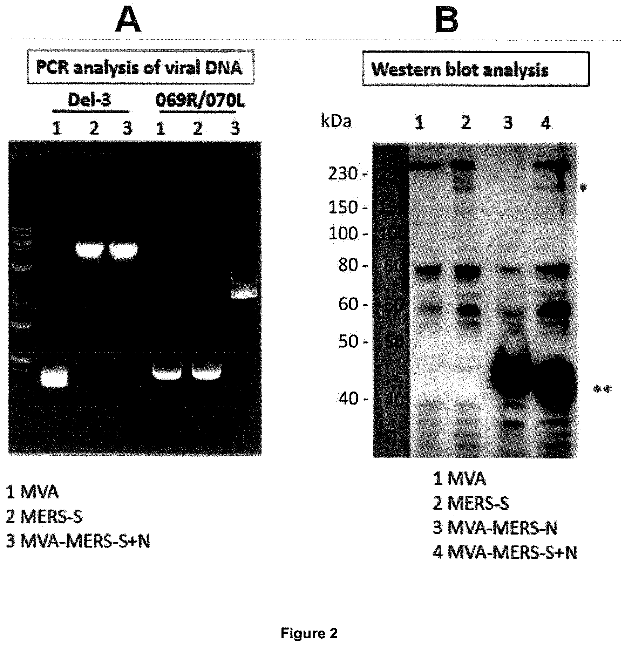

**Figure 2****: Characterization of recombinant MVA-MERS-S+N. (A)** PCR analysis of viral DNA to confirm the genetic integrity and genetic stability of final recombinant virus MVA-MERS-S+N. Genomic DNA preparations of non-recombinant MVA (MVA; (1)) or recombinant MVA-MERS-S (MERS-S; (2)) or double recombinant MVA-MERS-S+N (3) were used. Oligonucleotide primer pairs served to amplify DNA fragments specific for insertions into the sites of deletion III (Del-3) or 069R/070L. The PCR products obtained with MVA-MERS-S+N (3) template DNA confirm the precisely targeted insertion of recombinant S and N gene sequences, the proper removal of the marker gene *gfp*, and the absence of genomic DNA from non-recombinant MVA or single recombinant MVA-MERS-S. **(B)** Western blot analysis of protein lysates from infected Vero cells with MERS-CoV specific polyclonal antibodies reveals the co-synthesis of the about 210-kDa S protein (*) and the about 45-kDa N protein (**) upon infection with MVA-MERS-S+N (4). Lysates from Vero cells infected with single recombinant viruses MVA-MERS-S (MERS-S, (2)) and MVA-MERS-N (3) or infected with non-recombinant MVA (1) served as controls.

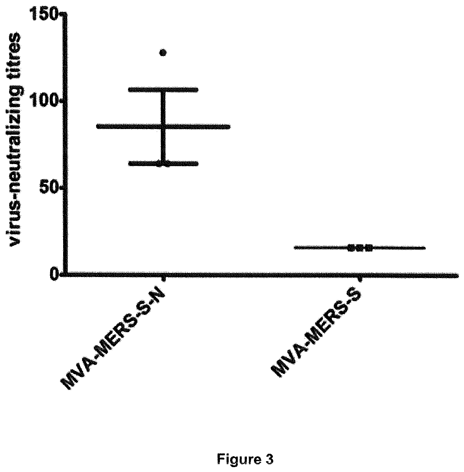

**Figure 3****: Enhanced immunogenicity of MVA-MERS-S+N.** Sera from BALB/c mice vaccinated with MVA-MERS-S+N demonstrate higher activities to neutralize MERS-CoV in tissue culture infections as compared to sera from vaccinated with single recombinant virus MVA-MERS-S. Mice vaccinated with non-recombinant MVA or saline served as controls and showed no neutralization activity (data not shown). All vaccines were given as a single intramuscular inoculation of 10⁸ plaque-forming-units of virus in physiological saline.

The examples illustrate the invention:

### Example 1: Methods and Materials

### Cells and virus

Vero cells (ATCC CCL-81) were grown in DMEM supplemented with 2mM L-glutamine 10% fetal calf serum (FCS). Modified Vaccinia virus Ankara (MVA, clonal isolate F6) and recombinant MVA were propagated on primary or secondary chicken embryo fibroblasts (CEF) prepared from 11-day-old chicken embryos (VALO BioMedia GmbH; eggs from specified pathogen free chicken flocks). CEF were grown in VP-SFM medium (Gibco) under serum free conditions. MVA-MERS-S is a recombinant Modified Vaccinia virus Ankara (MVA) expressing the spike protein of Middle East Respiratory Syndrome Coronavirus (MERS-CoV) (Song, 2013). CEF were used to titrate the infectivity of non-recombinant or recombinant MVA viruses. Titers were determined by plaque assay as described in Kremer M. et al. 2012 and values were reported in plaque forming units (PFU).

### Mice.

Female BALB/c mice (6 to 10 weeks old) were purchased from Charles River Laboratories (Sulzfeld, Germany). Intramuscular (i.m.) vaccinations were performed by injection of 50µl of virus suspension containing 10⁸ pfu of MVA, MVA-MERS-S, or MVA-MERS-S+N into the left hind leg. Injections of 50µl of PBS served as further control. For experimental work mice were housed in an Isocage unit (Tecniplast, Germany) and had free access to food and water.

### Generation of MVA-MERS-S+N

The full length nucleocapsid (N) protein gene sequence (1242 bp) of MERS-CoV 2012 (GenBank accession no. JX869059), flanked by BamHI and Notl restriction sites, was generated by DNA synthesis (Invitrogen Life Technology, Germany) and modified by codon optimization (MERS-N). MERS-N was then used to generate the MVA transfer vector plasmid pMERS-N which directs the insertion of MERS-N under the transcriptional control of the vaccinia virus early/late promoter PmH5 into the intergenic site 069R-070L within the MVA genome.

MVA expressing both MERS-S and MERS-N (MVA-MERS-S+N) was obtained by homologous recombination, as described previously (Kremer M, et al. 2012). Briefly, MVA-MERS-S+N formed in CEF monolayers (grown in 6-well tissue culture plates) infected with MVA-MERS-S (at moi 0.01 pfu) and transfected with 1 µg DNA/well of plasmid pMERS-N. After 48 hours cultures from transfection/infection were harvested, freeze-thawed twice and used for reinfection of 6-well CEF monolayers in log10 dilutions. Co-expression of the fluorescent marker protein GFP (under control of the vaccinia virus late promoter P11) was used to isolate clonal recombinant viruses MVA-MERS-S+N by screening for fluorescent cell foci during repetitive plaque purification. Virus stocks and vaccine preparations were amplified on CEF cells grown under serum free conditions.

### Characterization of recombinant MVA

CEF cells were infected with wild type or recombinant MVA (MVA, MVA-MERS-S or MVA-MERS-S+N, MOI 1). After 24h, total DNA was extracted with QIAamp DNA Mini Kit (QIAGEN, Germany). Elimination of wild-type MVA and correct insertion of the gene of interest within the MVA genome were controlled using PCR and two different primer sets for amplification of DNA fragments specifying (i) the deletion III site including the S gene, and (ii) the 069R/070L intergenic insertion site including the N gene. Primers used were (i) MVA_Del3-F (5'- GATGAGTGTAGATGCTGTTATTTTG (SEQ ID NO:5)) and MVA_Del3-R (5'-GCAGCTAAAAGAATAATGGAATTG (SEQ ID NO:6)), (ii) MVA_069R (5'- ATTCTCGCTGAGGAG TTGG (SEQ ID NO:7)) and MVA_070L (5'- GTCGTGTCTACAAAAGGAG (SEQ ID NO:8)). The synthesis of S and N proteins upon infection with MVA-MERS-S+N was assessed by Western blotting. Monolayers of Vero cells were separately infected with MVA, MVA-MERS-N, MVA-MERS-S or MVA-MERS-S+N. After 24 hours, cells were harvested and cell lysates were prepared for analysis by SDS-PAGE and immunoblotting using MERS-CoV specific polyclonal antibodies. Polyclonal antibodies from an MERS CoV infected cynomolgous macaque were used at 1:1000 dilution as MERS-CoV antigen specific antibodies. Lysates from Vero cells infected with single recombinant viruses MVA-MERS-S and MVA-MERS-N or infected with non-recombinant MVA served as controls.

### Results

The analysis of genomic viral DNA from MVA-MERS-S+N confirmed the precisely targeted and stable insertion of recombinant S and N gene sequences, the proper removal of the marker gene gfp, and the absence of genomic DNA from non-recombinant MVA or from single recombinant virus MVA-MERS-S (Figure 2A). Importantly, MVA-MERS-S+N allowed for co-synthesis of the MERS-CoV target antigens S and N. Western blot analysis of protein lysates from MVA-MERS-S+N infected cells clearly revealed the production of the about 210-kDa S protein and the about 45-kDa N protein upon infection with MVA-MERS-S+N (Figure 2B). Both recombinant proteins are expressed in MVA-MERS-S+N by the identical viral promoter (PmH5) for transcription regulation and therefore to quantitatively very comparable levels. Surprisingly, the MERS-CoV specific antibodies in the macaque serum preferentially recognize the N antigen. This data suggests that this MERS-CoV antigen is highly immunogenic because large amounts of N antigen-specific antibodies were apparently elicited upon infection.

Moreover, sera from BALB/c mice vaccinated with MVA-MERS-S+N demonstrate higher activities to neutralize MERS-CoV in tissue culture infections as compared to sera from vaccinated with single recombinant virus MVA-MERS-S (Figure 3). Briefly, groups of mice (n=3) were vaccinated by a single intramuscular inoculations of 10⁸ plaque-forming-units of each virus in physiological saline. Vaccine preparations with non-recombinant MVA or saline only served as controls. The capacity of MVA-MERS-S+N to induce higher levels of MERS-CoV neutralizing antibodies suggests that the co-delivery of the N antigen also enhances the immunogenicity of the MERS-S antigen.

### Further references

Arimilli D, Johnson JB, Clark KM, Graff AH, Alexander-Miller MA, Mizel SB, and Parks GD. 2008. Engineered Expression of the TLR5 Ligand Flagellin Enhances Paramyxovirus Activation of Human Dendritic Cell Function. J. Virol. 82(22):10975. DOI: 10.1128/JVI.01288-08.
Berchtold C, Panthel K, Jellbauer S, Köhn B, Roider E, Partilla M, Heesemann J, Endres S, Bourquin C and Rüssmann H. 2009. Superior Protective Immunity against Murine Listeriosis by Combined Vaccination with CpG DNA and Recombinant Salmonella enterica Serovar Typhimurium. Infect. Immun. 77(12):5501. DOI: 10.1128/IAI.00700-09.
Bermingham A, Brown MA, Aarons E, Tong C, Langrish C, Hoschler K, Brown K, Galiano M, Myers R, Pebody RG, Green HK, Boddington NL, Gopal R, Price N, Newsholme W, Drosten C, Fouchier RA, Zambon M. 2012. Severe respiratory illness caused by a novel coronavirus, in a patient transferred to United Kingdom from the Middle East. Euro Surveill. 17: pii_20290.
Coleman CM, Liu YV, Mu H, Taylor JK, Massare M, Flyer DC, Glenn GM, Smith GE, Frieman MB. 2014. Purified coronavirus spike protein nanoparticles induce coronavirus neutralizing antibodies in mice. Vaccine 32:3169-3174;
de Groot RJ, Baker SC, Baric RS, Brown CS, Drosten C, Enjuanes L, Fouchier RA, Galiano M, Gorbalenya AE, Memish ZA, Perlman S, Poon LL, Snijder EJ, Stephens GM, Woo PC, Zaki AM, Zambon M, Ziebuhr J. Middle East respiratory syndrome coronavirus (MERS-CoV): announcement of the Coronavirus Study Group. J Virol. 2013 Jul;87(14):7790-2. doi: 10.1128/JVI.01244-13. Epub 2013 May 15
Drexler I, Heller K, Ohlmann M, Erfle V, Sutter G. Recombinant Vaccinia Virus MVA for Generation and Analysis of T Cell Responses Against Tumor Associated Antigens. Methods Mol Med. 2000;35:57-73;
Gilbert SC. 2013. Clinical development of modified vaccinia virus Ankara vaccines. Vaccine 31:4241-4246.
Kim E, Okada K, Kenniston T, Raj VS, AlHajri MM, Farag EA, AlHajri F, Osterhaus AD, Haagmans BL, Gambotto A. 2014. Immunogenicity of an adenoviral-based Middle East Respiratory Syndrome coronavirus vaccine in BALB/c mice. Vaccine 32: 5975-5982
Kremer M, Volz A, Kreijtz JCM, Fux R, Lehmann M, Sutter G. 2012. Easy and efficient protocols for working with recombinant vaccinia virus MVA. Methods Mol. Biol. 890:59-92.
Ma C, Li Y, Wang L, Zhao G, Tao X, Tseng CT, Zhou Y, Du L, Jiang S. 2014. Intranasal vaccination with recombinant receptor-binding domain of MERS-CoV spike protein induces much stronger local mucosal immune responses than subcutaneous immunization: Implication for designing novel mucosal MERS vaccines. Vaccine 32:2100-2108;
Masters & Perlman "Coronaviridae", p. 825-858, Fields Virology, 6th Edition 2013, Knipe DM, Howley PM Eds, Lippincott Williams & Wilkins, Philadelphia.
Mayr A, Munz E. 1964. Veränderung von Vaccinevirus durch Dauerpassagen in Hühnerembryofibroblastenkulturen. Zentralbl. Bakteriol. B. 195: 24-35.
McBride R, van Zyl M, Fielding BC. The coronavirus nucleocapsid is a multifunctional protein. Viruses. 2014 Aug 7;6(8):2991-3018. doi: 10.3390/v6082991.
Meyer H, Sutter G, Mayr A. 1991. Mapping of deletions in the genome of the highly attenuated vaccinia virusMVAand their influence on virulence. J. Gen. Virol. 72:1031-1038.
Müller MA, Raj VS, Muth D, Meyer B, Kallies S, Smits SL, Wollny R, Bestebroer TM, Specht S, Suliman T, Zimmermann K, Binger T, Eckerle I, Tschapka M, Zaki AM, Osterhaus AD, Fouchier RA, Haagmans BL, Drosten C. Human coronavirus EMC does not require the SARS-coronavirus receptor and maintains broad replicative capability in mammalian cell lines. MBio. 2012 Dec 11;3(6). pii: e00515-12. doi: 10.1128/mBio.00515-12.
Song F, Fux R, Provacia LB, Volz A, Eickmann M, Becker S, Osterhaus ADME, Haagmans BL, Sutter G. J. Virol. 2013. Middle East Respiratory Syndrome Coronavirus Spike Protein Delivered by Modified Vaccinia Virus Ankara Efficiently Induces Virus-Neutralizing Antibodies J Virol. 87 (21): 11950-11954
Stittelaar KJ, Wyatt LS, de Swart RL, Vos HW, Groen J, van Amerongen G, van Binnendijk RS, Rozenblatt S, Moss B, Osterhaus AD. 2000. Protective immunity in macaques vaccinated with a modified vaccinia virus Ankara-based measles virus vaccine in the presence of passively acquired antibodies. J Virol. May;74(9):4236-43.
Scallana CD, Tingleya DW, Lindblooma JD, Toomeyb JS, and Tuckera SN. 2013. An Adenovirus-Based Vaccine with a Double-Stranded RNA Adjuvant Protects Mice and Ferrets against H5N1 Avian Influenza in Oral Delivery Models. Clin Vaccine Immunol. January vol. 20(1): 85-94.
Staib C, Drexler I, Sutter G. Construction and isolation of recombinant MVA. Methods Mol Biol. 2004;269:77-100;
Sutter G, Moss B. 1992. Nonreplicating vaccinia vector efficiently expresses recombinant genes. Proc. Natl. Acad. Sci. U. S. A. 89:10847-10851.
van Boheemen S, de Graaf M, Lauber C, Bestebroer TM, Raj VS, Zaki AM, Osterhaus AD, Haagmans BL, Gorbalenya AE, Snijder EJ, Fouchier RA. 2012. Genomic characterization of a newly discovered coronavirus associated with acute respiratory distress syndrome in humans. mBio 3(6):e00473-12. doi:10.1128/mBio.00473-12.
Volz A, Sutter G. 2013. Protective efficacy of modified vaccinia virus Ankara in preclinical studies. Vaccine 31:4235-4240.
Wyatt LS, Shors ST, Murphy BR, Moss B. Development of a replication-deficient recombinant vaccinia virus vaccine effective against parainfluenza virus 3 infection in an animal model. Vaccine. 1996 Oct; 14(15):1451-8
Zaki AM, van Boheemen S, Bestebroer TM, Osterhaus AD, Fouchier RAM. 2012. Isolation of a novel coronavirus from a man with pneumonia in Saudi Arabia. N. Engl. J. Med. 367:1814-1820.

## Claims

1. An immunogenic composition comprising the MERS-CoV N nucleocapsid protein or a nucleic acid molecule encoding the MERS-CoV N nucleocapsid protein, wherein the composition further comprises at least one additional immunogenic compound and wherein the additional immunogenic compound is the MERS-CoV S spike protein.

2. A vector comprising a nucleic acid molecule encoding the MERS-CoV N nucleocapsid protein for use as a vaccine, wherein the vector further comprises at least one additional nucleic acid molecule encoding an immunogenic compound and wherein the additional immunogenic compound is the MERS-CoV S spike protein.

3. The vector for use of claim 2, wherein the vector is selected from the group consisting of vaccinia virus, avipoxvirus, adenovirus, alphavirus, rhabdovirus, and herpesvirus.

4. The vector for use of claim 3, wherein the vector is a vaccinia virus.

5. The vector for use of claim 4, wherein the vaccinia virus is a modified vaccinia virus Ankara (MVA).

6. The vector for use of any one of claims 2 to 5, wherein the vector further comprises at least one additional nucleic acid molecule encoding a compound selected from the group consisting of immunogenic compounds and adjuvants.

7. The immunogenic composition of claim 1, or the vector for use of any one of claims 2 to 6, wherein the MERS-CoV N nucleocapsid protein comprises or consists of the amino acid sequence of SEQ ID NO:1 or a functional variant thereof, wherein the functional variant has at least 95% sequence identity to the amino acid sequence of SEQ ID NO:1 and retains the immunogenic activity of the MERS-CoV N nucleocapsid protein consisting of SEQ ID NO:1.

8. The immunogenic composition of any one of claims 1 or 7, or the vector for use of any one of claims 3 to 7, wherein the MERS-CoV S spike protein comprises or consists of the amino acid sequence of SEQ ID NO:3 or a functional variant thereof, wherein the functional variant has at least 95% sequence identity to the amino acid sequence of SEQ ID NO:3 and retains the immunogenic activity of the MERS-CoV S spike protein consisting of SEQ ID NO:3.

9. The immunogenic composition of claim 1, 7 or 8, or the vector of any one of claims 2 to 8, for use in inducing a protective immune response against MERS-CoV.

## Patentansprüche

1. Immunogene Zusammensetzung, umfassend das MERS-CoV N Nukleokapsidprotein oder ein Nukleinsäuremolekül, das das MERS-CoV N Neukleokapsidprotein kodiert, wobei die Zusammensetzung ferner mindestens eine zusätzliche immunogene Verbindung umfasst, und wobei die zusätzliche immunogene Verbindung das MERS-CoV S Spike-Protein ist.

2. Vektor, umfassend ein Nukleinsäuremolekül, das das MERS-CoV N Nukleokapsidprotein kodiert, für die Verwendung als Vakzin, wobei der Vektor ferner mindestens ein zusätzliches Nukleinsäuremolekül umfasst, das eine immunogene Verbindung kodiert, und wobei die zusätzliche immunogene Verbindung das MERS-CoV S Spike-Protein ist.

3. Vektor für die Verwendung nach Anspruch 2, wobei der Vektor ausgewählt ist aus der Gruppe bestehend aus Vacciniavirus, Avipoxvirus, Adenovirus, Alphavirus, Rhabdovirus und Herpesvirus.

4. Vektor für die Verwendung nach Anspruch 3, wobei der Vektor ein Vacciniavirus ist.

5. Vektor für die Verwendung nach Anspruch 4, wobei das Vacciniavirus ein modifizierter Ankara-Vacciniavirus (MVA) ist.

6. Vektor für die Verwendung nach einem der Ansprüche 2 bis 5, wobei der Vektor ferner mindestens ein zusätzliches Nukleinsäuremolekül umfasst, das eine Verbindung kodiert, die ausgewählt ist aus immunogenen Verbindungen und Adjuvanzien.

7. Immunogene Zusammensetzung nach Anspruch 1, oder Vektor für die Verwendung nach einem der Ansprüche 2 bis 6, wobei das MERS-CoV N Nukleokapsidprotein die Aminosäuresequenz der SEQ ID Nr. 1 oder eine funktionelle Variante davon umfasst oder aus der Aminosäuresequenz der SEQ ID Nr. 1 oder einer funktionellen Variante davon besteht, wobei die funktionelle Variante mindestens 95% Sequenzidentität zur Aminosäuresequenz der SEQ ID Nr. 1 hat und die immunogene Aktivität des MERS-CoV N Nukleokapsidproteins, das aus SEQ ID Nr. 1 besteht, behält.

8. Immunogene Zusammensetzung nach einem der Ansprüche 1 oder 7, oder Vektor für die Verwendung nach einem der Ansprüche 3 bis 7, wobei das MERS-CoV S Spike-Protein die Aminosäuresequenz der SEQ ID Nr. 3 oder eine funktionelle Variante davon umfasst oder aus der Aminosäuresequenz der SEQ ID Nr. 3 oder einer funktionellen Variante davon besteht, wobei die funktionelle Variante mindestens 95% Sequenzidentität zur Aminosäuresequenz der SEQ ID Nr. 3 hat und die immunogene Aktivität des MERS-CoV S Spike-Proteins, das aus SEQ ID Nr. 3 besteht, behält.

9. Immunogene Zusammensetzung nach Anspruch 1, 7 oder 8 oder Vektor nach einem der Ansprüche 2 bis 8, für die Verwendung zum Induzieren einer protektiven Immunantwort gegen MERS-CoV.

## Revendications

1. Composition immunogène comprenant la protéine de nucléocapside N du MERS-CoV ou une molécule d'acide nucléique codant pour la protéine de nucléocapside N du MERS-CoV, dans laquelle la composition comprend en outre au moins un composé immunogène supplémentaire et dans laquelle le composé immunogène supplémentaire est la protéine spiculaire S du MERS-CoV.

2. Vecteur comprenant une molécule d'acide nucléique codant pour la protéine de nucléocapside N du MERS-CoV pour une utilisation à titre de vaccin, dans laquelle le vecteur comprend en outre au moins une molécule d'acide nucléique supplémentaire codant pour un composé immunogène et dans laquelle le composé immunogène supplémentaire est la protéine spiculaire S du MERS-CoV.

3. Vecteur pour son utilisation selon la revendication 2, dans laquelle le vecteur est choisi dans le groupe constitué par le virus de la vaccine, le virus de la variole aviaire, un adénovirus, un alphavirus, un rhabdovirus, et un herpèsvirus.

4. Vecteur pour son utilisation selon la revendication 3, dans laquelle le vecteur est un virus de la vaccine.

5. Vecteur pour son utilisation selon la revendication 4, dans laquelle le vecteur de la vaccine est un virus modifié de la vaccine Ankara (MVA).

6. Vecteur pour son utilisation selon l'une quelconque des revendications 2 à 5, dans laquelle le vecteur comprend en outre au moins une molécule d'acide nucléique supplémentaire codant pour un composé choisi dans le groupe constitué par les composés immunogènes et les adjuvants.

7. Composition immunogène selon la revendication 1, ou vecteur pour son utilisation selon l'une quelconque des revendications 2 à 6, dans laquelle la protéine de nucléocapside N du MERS-CoV comprend ou est constituée par la séquence d'acides aminés de SEQ ID NO: 1 ou un variant fonctionnel de celle-ci, où le variant fonctionnel a une identité de séquence d'au moins 95 % avec la séquence d'acides aminés de SEQ ID NO: 1 et conserve l'activité immunogène de la protéine de nucléocapside N du MERS-CoV constituée par SEQ ID NO: 1.

8. Composition immunogène selon l'une quelconque des revendications 1 ou 7, ou vecteur pour son utilisation selon l'une quelconque des revendications 3 à 7, dans laquelle la protéine spiculaire S du MERS-CoV comprend ou est constituée par la séquence d'acides aminés de SEQ ID NO: 3 ou un variant fonctionnel de celle-ci, où le variant fonctionnel a une identité de séquence d'au moins 95 % avec la séquence d'acides aminés de SEQ ID NO: 3 et conserve l'activité immunogène de la protéine spiculaire S du MERS-CoV constituée par SEQ ID NO: 3.

9. Composition immunogène selon la revendication 1, 7 ou 8, ou vecteur selon l'une quelconque des revendications 2 à 8, pour son utilisation dans l'induction d'une réponse immunitaire protectrice contre le MERS-CoV.


### SEQUENCE LISTING

<110> Ludwig-Maximilians-universität München
   Philipps-Universität Marburg
<120> A Novel vaccine against the Middle East Respiratory Syndrome Coronavirus
   (MERS-CoV)
<130> X2872 EP
<160> 14
<170> BiSSAP 1.2
<210> 1
   <211> 413
   <212> PRT
   <213> Human betacoronavirus 2c EMC/2012
<220>
   <223> N protein
<400> 1
<210> 2
   <211> 1242
   <212> DNA
   <213> Human betacoronavirus 2c EMC/2012
<220>
   <221> source
   <222> 1..1242
   <223> /mol_type="unassigned DNA" /note="N protein" /organism="Human betacoronavirus 2c EMC/2012"
<400> 2
   atggcatccc ctgctgcacc tcgtgctgtt tcctttgccg ataacaatga tataacaaat      60
   acaaacctat ctcgaggtag aggacgtaat ccaaaaccac gagctgcacc aaataacact     120
   gtctcttggt acactgggct tacccaacac gggaaagtcc ctcttacctt tccacctggg     180
   cagggtgtac ctcttaatgc caattctacc cctgcgcaaa atgctgggta ttggcggaga     240
   caggacagaa aaattaatac cgggaatgga attaagcaac tggctcccag gtggtacttc     300
   tactacactg gaactggacc cgaagcagca ctcccattcc gggctgttaa ggatggcatc     360
   gtttgggtcc atgaagatgg cgccactgat gctccttcaa cttttgggac gcggaaccct     420
   aacaatgatt cagctattgt tacacaattc gcgcccggta ctaagcttcc taaaaacttc     480
   cacattgagg ggactggagg caatagtcaa tcatcttcaa gagcctctag cttaagcaga     540
   aactcttcca gatctagttc acaaggttca agatcaggaa actctacccg cggcacttct     600
   ccaggtccat ctggaatcgg agcagtagga ggtgatctac tttaccttga tcttctgaac     660
   agactacaag cccttgagtc tggcaaagta aagcaatcgc agccaaaagt aatcactaag     720
   aaagatgctg ctgctgctaa aaataagatg cgccacaagc gcacttccac caaaagtttc     780
   aacatggtgc aagcttttgg tcttcgcgga ccaggagacc tccagggaaa ctttggtgat     840
   cttcaattga ataaactcgg cactgaggac ccacgttggc cccaaattgc tgagcttgct     900
   cctacagcca gtgcttttat gggtatgtcg caatttaaac ttacccatca gaacaatgat     960
   gatcatggca accctgtgta cttccttcgg tacagtggag ccattaaact tgacccaaag    1020
   aatcccaact acaataagtg gttggagctt cttgagcaaa atattgatgc ctacaaaacc    1080
   ttccctaaga aggaaaagaa acaaaaggca ccaaaagaag aatcaacaga ccaaatgtct    1140
   gaacctccaa aggagcagcg tgtgcaaggt agcatcactc agcgcactcg cacccgtcca    1200
   agtgttcagc ctggtccaat gattgatgtt aacactgatt ag                        1242
<210> 3
   <211> 1353
   <212> PRT
   <213> Human betacoronavirus 2c EMC/2012
<220>
   <223> S protein
<400> 3
   Met Ile His Ser Val Phe Leu Leu Met Phe Leu Leu Thr Pro Thr Glu
   1               5                   10                  15
   Ser Tyr Val Asp Val Gly Pro Asp Ser Val Lys Ser Ala Cys Ile Glu
               20                  25                  30
   Val Asp Ile Gln Gln Thr Phe Phe Asp Lys Thr Trp Pro Arg Pro Ile
           35                  40                  45
   Asp Val Ser Lys Ala Asp Gly Ile Ile Tyr Pro Gln Gly Arg Thr Tyr
       50                  55                  60
   Ser Asn Ile Thr Ile Thr Tyr Gln Gly Leu Phe Pro Tyr Gln Gly Asp
   65                  70                  75                  80
   His Gly Asp Met Tyr Val Tyr Ser Ala Gly His Ala Thr Gly Thr Thr
                   85                  90                  95
   Pro Gln Lys Leu Phe Val Ala Asn Tyr Ser Gln Asp Val Lys Gln Phe
               100                 105                 110
   Ala Asn Gly Phe Val Val Arg Ile Gly Ala Ala Ala Asn Ser Thr Gly
           115                 120                 125
   Thr Val Ile Ile Ser Pro Ser Thr Ser Ala Thr Ile Arg Lys Ile Tyr
       130                 135                 140
   Pro Ala Phe Met Leu Gly Ser Ser Val Gly Asn Phe Ser Asp Gly Lys
   145                 150                 155                 160
   Met Gly Arg Phe Phe Asn His Thr Leu Val Leu Leu Pro Asp Gly Cys
                   165                 170                 175
   Gly Thr Leu Leu Arg Ala Phe Tyr Cys Ile Leu Glu Pro Arg Ser Gly
               180                 185                 190
   Asn His Cys Pro Ala Gly Asn Ser Tyr Thr Ser Phe Ala Thr Tyr His
           195                 200                 205
   Thr Pro Ala Thr Asp Cys Ser Asp Gly Asn Tyr Asn Arg Asn Ala Ser
       210                 215                 220
   Leu Asn Ser Phe Lys Glu Tyr Phe Asn Leu Arg Asn Cys Thr Phe Met
   225                 230                 235                 240
   Tyr Thr Tyr Asn Ile Thr Glu Asp Glu Ile Leu Glu Trp Phe Gly Ile
                   245                 250                 255
   Thr Gln Thr Ala Gln Gly Val His Leu Phe Ser Ser Arg Tyr Val Asp
               260                 265                 270
   Leu Tyr Gly Gly Asn Met Phe Gln Phe Ala Thr Leu Pro Val Tyr Asp
           275                 280                 285
   Thr Ile Lys Tyr Tyr Ser Ile Ile Pro His Ser Ile Arg Ser Ile Gln
       290                 295                 300
   Ser Asp Arg Lys Ala Trp Ala Ala Phe Tyr Val Tyr Lys Leu Gln Pro
   305                 310                 315                 320
   Leu Thr Phe Leu Leu Asp Phe Ser Val Asp Gly Tyr Ile Arg Arg Ala
                   325                 330                 335
   Ile Asp Cys Gly Phe Asn Asp Leu Ser Gln Leu His Cys Ser Tyr Glu
               340                 345                 350
   Ser Phe Asp Val Glu Ser Gly Val Tyr Ser Val Ser Ser Phe Glu Ala
           355                 360                 365
   Lys Pro Ser Gly Ser Val Val Glu Gln Ala Glu Gly Val Glu Cys Asp
       370                 375                 380
   Phe Ser Pro Leu Leu Ser Gly Thr Pro Pro Gln Val Tyr Asn Phe Lys
   385                 390                 395                 400
   Arg Leu Val Phe Thr Asn Cys Asn Tyr Asn Leu Thr Lys Leu Leu Ser
                   405                 410                 415
   Leu Phe Ser Val Asn Asp Phe Thr Cys Ser Gln Ile Ser Pro Ala Ala
               420                 425                 430
   Ile Ala Ser Asn Cys Tyr Ser Ser Leu Ile Leu Asp Tyr Phe Ser Tyr
           435                 440                 445
   Pro Leu Ser Met Lys Ser Asp Leu Ser Val Ser Ser Ala Gly Pro Ile
       450                 455                 460
   Ser Gln Phe Asn Tyr Lys Gln Ser Phe Ser Asn Pro Thr Cys Leu Ile
   465                 470                 475                 480
   Leu Ala Thr Val Pro His Asn Leu Thr Thr Ile Thr Lys Pro Leu Lys
                   485                 490                 495
   Tyr Ser Tyr Ile Asn Lys Cys Ser Arg Leu Leu Ser Asp Asp Arg Thr
               500                 505                 510
   Glu Val Pro Gln Leu Val Asn Ala Asn Gln Tyr Ser Pro Cys Val Ser
           515                 520                 525
   Ile Val Pro Ser Thr Val Trp Glu Asp Gly Asp Tyr Tyr Arg Lys Gln
       530                 535                 540
   Leu Ser Pro Leu Glu Gly Gly Gly Trp Leu Val Ala Ser Gly Ser Thr
   545                 550                 555                 560
   Val Ala Met Thr Glu Gln Leu Gln Met Gly Phe Gly Ile Thr Val Gln
                   565                 570                 575
   Tyr Gly Thr Asp Thr Asn Ser Val Cys Pro Lys Leu Glu Phe Ala Asn
               580                 585                 590
   Asp Thr Lys Ile Ala Ser Gln Leu Gly Asn Cys Val Glu Tyr Ser Leu
           595                 600                 605
   Tyr Gly Val Ser Gly Arg Gly Val Phe Gln Asn Cys Thr Ala Val Gly
       610                 615                 620
   Val Arg Gln Gln Arg Phe Val Tyr Asp Ala Tyr Gln Asn Leu Val Gly
   625                 630                 635                 640
   Tyr Tyr Ser Asp Asp Gly Asn Tyr Tyr Cys Leu Arg Ala Cys Val Ser
                   645                 650                 655
   Val Pro Val Ser Val Ile Tyr Asp Lys Glu Thr Lys Thr His Ala Thr
               660                 665                 670
   Leu Phe Gly Ser Val Ala Cys Glu His Ile Ser Ser Thr Met Ser Gln
           675                 680                 685
   Tyr Ser Arg Ser Thr Arg Ser Met Leu Lys Arg Arg Asp Ser Thr Tyr
       690                 695                 700
   Gly Pro Leu Gln Thr Pro Val Gly Cys Val Leu Gly Leu Val Asn Ser
   705                 710                 715                 720
   Ser Leu Phe Val Glu Asp Cys Lys Leu Pro Leu Gly Gln Ser Leu Cys
                   725                 730                 735
   Ala Leu Pro Asp Thr Pro Ser Thr Leu Thr Pro Arg Ser Val Arg Ser
               740                 745                 750
   Val Pro Gly Glu Met Arg Leu Ala Ser Ile Ala Phe Asn His Pro Ile
           755                 760                 765
   Gln Val Asp Gln Leu Asn Ser Ser Tyr Phe Lys Leu Ser Ile Pro Thr
       770                 775                 780
   Asn Phe Ser Phe Gly Val Thr Gln Glu Tyr Ile Gln Thr Thr Ile Gln
   785                 790                 795                 800
   Lys Val Thr Val Asp Cys Lys Gln Tyr Val Cys Asn Gly Phe Gln Lys
                   805                 810                 815
   Cys Glu Gln Leu Leu Arg Glu Tyr Gly Gln Phe Cys Ser Lys Ile Asn
               820                 825                 830
   Gln Ala Leu His Gly Ala Asn Leu Arg Gln Asp Asp Ser Val Arg Asn
           835                 840                 845
   Leu Phe Ala Ser Val Lys Ser Ser Gln Ser Ser Pro Ile Ile Pro Gly
       850                 855                 860
   Phe Gly Gly Asp Phe Asn Leu Thr Leu Leu Glu Pro Val Ser Ile Ser
   865                 870                 875                 880
   Thr Gly Ser Arg Ser Ala Arg Ser Ala Ile Glu Asp Leu Leu Phe Asp
                   885                 890                 895
   Lys Val Thr Ile Ala Asp Pro Gly Tyr Met Gln Gly Tyr Asp Asp Cys
               900                 905                 910
   Met Gln Gln Gly Pro Ala Ser Ala Arg Asp Leu Ile Cys Ala Gln Tyr
           915                 920                 925
   Val Ala Gly Tyr Lys Val Leu Pro Pro Leu Met Asp Val Asn Met Glu
       930                 935                 940
   Ala Ala Tyr Thr Ser Ser Leu Leu Gly Ser Ile Ala Gly Val Gly Trp
   945                 950                 955                 960
   Thr Ala Gly Leu Ser Ser Phe Ala Ala Ile Pro Phe Ala Gln Ser Ile
                   965                 970                 975
   Phe Tyr Arg Leu Asn Gly Val Gly Ile Thr Gln Gln Val Leu Ser Glu
               980                 985                 990
   Asn Gln Lys Leu Ile Ala Asn Lys Phe Asn Gln Ala Leu Gly Ala Met
           995                 1000                1005
   Gln Thr Gly Phe Thr Thr Thr Asn Glu Ala Phe Gln Lys Val Gln Asp
       1010                1015                1020
   Ala Val Asn Asn Asn Ala Gln Ala Leu Ser Lys Leu Ala Ser Glu Leu
   1025                1030                1035                1040
   Ser Asn Thr Phe Gly Ala Ile Ser Ala Ser Ile Gly Asp Ile Ile Gln
                   1045                1050                1055
   Arg Leu Asp Val Leu Glu Gln Asp Ala Gln Ile Asp Arg Leu Ile Asn
               1060                1065                1070
   Gly Arg Leu Thr Thr Leu Asn Ala Phe Val Ala Gln Gln Leu Val Arg
           1075                1080                1085
   Ser Glu Ser Ala Ala Leu Ser Ala Gln Leu Ala Lys Asp Lys Val Asn
       1090                1095                1100
   Glu Cys Val Lys Ala Gln Ser Lys Arg Ser Gly Phe Cys Gly Gln Gly
   1105                1110                1115                1120
   Thr His Ile Val Ser Phe Val Val Asn Ala Pro Asn Gly Leu Tyr Phe
                   1125                1130                1135
   Met His Val Gly Tyr Tyr Pro Ser Asn His Ile Glu Val Val Ser Ala
               1140                1145                1150
   Tyr Gly Leu Cys Asp Ala Ala Asn Pro Thr Asn Cys Ile Ala Pro Val
           1155                1160                1165
   Asn Gly Tyr Phe Ile Lys Thr Asn Asn Thr Arg Ile Val Asp Glu Trp
       1170                1175                1180
   Ser Tyr Thr Gly Ser Ser Phe Tyr Ala Pro Glu Pro Ile Thr Ser Leu
   1185                1190                1195                1200
   Asn Thr Lys Tyr Val Ala Pro Gln Val Thr Tyr Gln Asn Ile Ser Thr
                   1205                1210                1215
   Asn Leu Pro Pro Pro Leu Leu Gly Asn Ser Thr Gly Ile Asp Phe Gln
               1220                1225                1230
   Asp Glu Leu Asp Glu Phe Phe Lys Asn Val Ser Thr Ser Ile Pro Asn
           1235                1240                1245
   Phe Gly Ser Leu Thr Gln Ile Asn Thr Thr Leu Leu Asp Leu Thr Tyr
       1250                1255                1260
   Glu Met Leu Ser Leu Gln Gln Val Val Lys Ala Leu Asn Glu Ser Tyr
   1265                1270                1275                1280
   Ile Asp Leu Lys Glu Leu Gly Asn Tyr Thr Tyr Tyr Asn Lys Trp Pro
                   1285                1290                1295
   Trp Tyr Ile Trp Leu Gly Phe Ile Ala Gly Leu Val Ala Leu Ala Leu
               1300                1305                1310
   Cys Val Phe Phe Ile Leu Cys Cys Thr Gly Cys Gly Thr Asn Cys Met
           1315                1320                1325
   Gly Lys Leu Lys Cys Asn Arg Cys Cys Asp Arg Tyr Glu Glu Tyr Asp
       1330                1335                1340
   Leu Glu Pro His Lys Val His Val His
   1345                1350
<210> 4
   <211> 4062
   <212> DNA
   <213> Human betacoronavirus 2c EMC/2012
<220>
   <221> source
   <222> 1..4062
   <223> /mol_type="unassigned DNA" /note="S protein" /organism="Human betacoronavirus 2c EMC/2012"
<400> 4
   atgatacact cagtgtttct actgatgttc ttgttaacac ctacagaaag ttacgttgat      60
   gtagggccag attctgttaa gtctgcttgt attgaggttg atatacaaca gactttcttt     120
   gataaaactt ggcctaggcc aattgatgtt tctaaggctg acggtattat ataccctcaa     180
   ggccgtacat attctaacat aactatcact tatcaaggtc tttttcccta tcagggagac     240
   catggtgata tgtatgttta ctctgcagga catgctacag gcacaactcc acaaaagttg     300
   tttgtagcta actattctca ggacgtcaaa cagtttgcta atgggtttgt cgtccgtata     360
   ggagcagctg ccaattccac tggcactgtt attattagcc catctaccag cgctactata     420
   cgaaaaattt accctgcttt tatgctgggt tcttcagttg gtaatttctc agatggtaaa     480
   atgggccgct tcttcaatca tactctagtt cttttgcccg atggatgtgg cactttactt     540
   agagcttttt attgtattct agagcctcgc tctggaaatc attgtcctgc tggcaattcc     600
   tatacttctt ttgccactta tcacactcct gcaacagatt gttctgatgg caattacaat     660
   cgtaatgcca gtctgaactc ttttaaggag tattttaatt tacgtaactg cacctttatg     720
   tacacttata acattaccga agatgagatt ttagagtggt ttggcattac acaaactgct     780
   caaggtgttc acctcttctc atctcggtat gttgatttgt acggcggcaa tatgtttcaa     840
   tttgccacct tgcctgttta tgatactatt aagtattatt ctatcattcc tcacagtatt     900
   cgttctatcc aaagtgatag aaaagcttgg gctgccttct acgtatataa acttcaaccg     960
   ttaactttcc tgttggattt ttctgttgat ggttatatac gcagagctat agactgtggt    1020
   tttaatgatt tgtcacaact ccactgctca tatgaatcct tcgatgttga atctggagtt    1080
   tattcagttt cgtctttcga agcaaaacct tctggctcag ttgtggaaca ggctgaaggt    1140
   gttgaatgtg atttttcacc tcttctgtct ggcacacctc ctcaggttta taatttcaag    1200
   cgtttggttt ttaccaattg caattataat cttaccaaat tgctttcact tttttctgtg    1260
   aatgatttta cttgtagtca aatatctcca gcagcaattg ctagcaactg ttattcttca    1320
   ctgattttgg attacttttc atacccactt agtatgaaat ccgatctcag tgttagttct    1380
   gctggtccaa tatcccagtt taattataaa cagtcctttt ctaatcccac atgtttgatt    1440
   ttagcgactg ttcctcataa ccttactact attactaagc ctcttaagta cagctatatt    1500
   aacaagtgct ctcgtcttct ttctgatgat cgtactgaag tacctcagtt agtgaacgct    1560
   aatcaatact caccctgtgt atccattgtc ccatccactg tgtgggaaga cggtgattat    1620
   tataggaaac aactatctcc acttgaaggt ggtggctggc ttgttgctag tggctcaact 1680
   gttgccatga ctgagcaatt acagatgggc tttggtatta cagttcaata tggtacagac 1740
   accaatagtg tttgccccaa gcttgaattt gctaatgaca caaaaattgc ctctcaatta 1800
   ggcaattgcg tggaatattc cctctatggt gtttcgggcc gtggtgtttt tcagaattgc 1860
   acagctgtag gtgttcgaca gcagcgcttt gtttatgatg cgtaccagaa tttagttggc 1920
   tattattctg atgatggcaa ctactactgt ttgcgtgctt gtgttagtgt tcctgtttct 1980
   gtcatctatg ataaagaaac taaaacccac gctactctat ttggtagtgt tgcatgtgaa 2040
   cacatttctt ctaccatgtc tcaatactcc cgttctacgc gatcaatgct taaacggcga 2100
   gattctacat atggccccct tcagacacct gttggttgtg tcctaggact tgttaattcc 2160
   tctttgttcg tagaggactg caagttgcct cttggtcaat ctctctgtgc tcttcctgac 2220
   acacctagta ctctcacacc tcgcagtgtg cgctctgttc caggtgaaat gcgcttggca 2280
   tccattgctt ttaatcatcc tattcaggtt gatcaactta atagtagtta ttttaaatta 2340
   agtataccca ctaatttttc ctttggtgtg actcaggagt acattcagac aaccattcag 2400
   aaagttactg ttgattgtaa acagtacgtt tgcaatggtt tccagaagtg tgagcaatta 2460
   ctgcgcgagt atggccagtt ttgttccaaa ataaaccagg ctctccatgg tgccaattta 2520
   cgccaggatg attctgtacg taatttgttt gcgagcgtga aaagctctca atcatctcct 2580
   atcataccag gttttggagg tgactttaat ttgacacttc tagaacctgt ttctatatct 2640
   actggcagtc gtagtgcacg tagtgctatt gaggatttgc tatttgacaa agtcactata 2700
   gctgatcctg gttatatgca aggttacgat gattgcatgc agcaaggtcc agcatcagct 2760
   cgtgatctta tttgtgctca atatgtggct ggttacaaag tattacctcc tcttatggat 2820
   gttaatatgg aagccgcgta tacttcatct ttgcttggca gcatagcagg tgttggctgg 2880
   actgctggct tatcctcctt tgctgctatt ccatttgcac agagtatctt ttataggtta 2940
   aacggtgttg gcattactca acaggttctt tcagagaacc aaaagcttat tgccaataag 3000
   tttaatcagg ctctgggagc tatgcaaaca ggcttcacta caactaatga agcttttcag 3060
   aaggttcagg atgctgtgaa caacaatgca caggctctat ccaaattagc tagcgagcta 3120
   tctaatactt ttggtgctat ttccgcctct attggagaca tcatacaacg tcttgatgtt 3180
   ctcgaacagg acgcccaaat agacagactt attaatggcc gtttgacaac actaaatgct 3240
   tttgttgcac agcagcttgt tcgttccgaa tcagctgctc tttccgctca attggctaaa 3300
   gataaagtca atgagtgtgt caaggcacaa tccaagcgtt ctggattttg cggtcaaggc 3360
   acacatatag tgtcctttgt tgtaaatgcc cctaatggcc tttacttcat gcatgttggt 3420
   tattacccta gcaaccacat tgaggttgtt tctgcttatg gtctttgcga tgcagctaac 3480
   cctactaatt gtatagcccc tgttaatggc tactttatta aaactaataa cactaggatt 3540
   gttgatgagt ggtcatatac tggctcgtcc ttctatgcac ctgagcccat tacctccctt 3600
   aatactaagt atgttgcacc acaggtgaca taccaaaaca tttctactaa cctccctcct 3660
   cctcttctcg gcaattccac cgggattgac ttccaagatg agttggatga gtttttcaaa 3720
   aatgttagca ccagtatacc taattttggt tccctaacac agattaatac tacattactc 3780
   gatcttacct acgagatgtt gtctcttcaa caagttgtta aagcccttaa tgagtcttac 3840
   atagacctta aagagcttgg caattatact tattacaaca aatggccgtg gtacatttgg 3900
   cttggtttca ttgctgggct tgttgcctta gctctatgcg tcttcttcat actgtgctgc 3960
   actggttgtg gcacaaactg tatgggaaaa cttaagtgta atcgttgttg tgatagatac 4020
   gaggaatacg acctcgagcc gcataaggtt catgttcact aa 4062
<210> 5
   <211> 25
   <212> DNA
   <213> Artificial Sequence
<220>
   <221> source
   <222> 1..25
   <223> /mol_type="unassigned DNA" /note="MVA_Del3-F primer" /organism="Artificial Sequence"
<400> 5
   gatgagtgta gatgctgtta ttttg 25
<210> 6
   <211> 24
   <212> DNA
   <213> Artificial Sequence
<220>
   <221> source
   <222> 1..24
   <223> /mol_type="unassigned DNA" /note="MVA_Del3-R primer" /organism="Artificial Sequence"
<400> 6
   gcagctaaaa gaataatgga attg 24
<210> 7
   <211> 19
   <212> DNA
   <213> Artificial Sequence
<220>
   <221> source
   <222> 1..19
   <223> /mol_type="unassigned DNA" /note="MVA_069R primer" /organism="Artificial Sequence"
<400> 7
   attctcgctg aggagttgg 19
<210> 8
   <211> 19
   <212> DNA
   <213> Artificial Sequence
<220>
   <221> source
   <222> 1..19
   <223> /mol_type="unassigned DNA" /note="MVA_070L primer" /organism="Artificial Sequence"
<400> 8
   gtcgtgtcta caaaaggag 19
<210> 9
   <211> 82
   <212> PRT
   <213> Human betacoronavirus 2c EMC/2012
<220>
   <223> E protein
<400> 9
   Met Leu Pro Phe Val Gln Glu Arg Ile Gly Leu Phe Ile Val Asn Phe
   1               5                   10                  15
   Phe Ile Phe Thr Val Val Cys Ala Ile Thr Leu Leu Val Cys Met Ala
               20                  25                  30
   Phe Leu Thr Ala Thr Arg Leu Cys Val Gln Cys Met Thr Gly Phe Asn
           35                  40                  45
   Thr Leu Leu Val Gln Pro Ala Leu Tyr Leu Tyr Asn Thr Gly Arg Ser
       50                  55                  60
   Val Tyr Val Lys Phe Gln Asp Ser Lys Pro Pro Leu Pro Pro Asp Glu
   65                  70                  75                  80
   Trp Val
<210> 10
   <211> 219
   <212> PRT
   <213> Human betacoronavirus 2c EMC/2012
<220>
   <223> M protein
<400> 10
   Met Ser Asn Met Thr Gln Leu Thr Glu Ala Gln Ile Ile Ala Ile Ile
   1               5                   10                  15
   Lys Asp Trp Asn Phe Ala Trp Ser Leu Ile Phe Leu Leu Ile Thr Ile
               20                  25                  30
   Val Leu Gln Tyr Gly Tyr Pro Ser Arg Ser Met Thr Val Tyr Val Phe
           35                  40                  45
   Lys Met Phe Val Leu Trp Leu Leu Trp Pro Ser Ser Met Ala Leu Ser
       50                  55                  60
   Ile Phe Ser Ala Val Tyr Pro Ile Asp Leu Ala Ser Gln Ile Ile Ser
   65                  70                  75                  80
   Gly Ile Val Ala Ala Val Ser Ala Met Met Trp Ile Ser Tyr Phe Val
                   85                  90                  95
   Gln Ser Ile Arg Leu Phe Met Arg Thr Gly Ser Trp Trp Ser Phe Asn
               100                 105                 110
   Pro Glu Thr Asn Cys Leu Leu Asn Val Pro Phe Gly Gly Thr Thr Val
           115                 120                 125
   Val Arg Pro Leu Val Glu Asp Ser Thr Ser Val Thr Ala Val Val Thr
       130                 135                 140
   Asn Gly His Leu Lys Met Ala Gly Met His Phe Gly Ala Cys Asp Tyr
   145                 150                 155                 160
   Asp Arg Leu Pro Asn Glu Val Thr Val Ala Lys Pro Asn Val Leu Ile
                   165                 170                 175
   Ala Leu Lys Met Val Lys Arg Gln Ser Tyr Gly Thr Asn Ser Gly Val
               180                 185                 190
   Ala Ile Tyr His Arg Tyr Lys Ala Gly Asn Tyr Arg Ser Pro Pro Ile
           195                 200                 205
   Thr Ala Asp Ile Glu Leu Ala Leu Leu Arg Ala
       210                 215
<210> 11
   <211> 103
   <212> PRT
   <213> Human betacoronavirus 2c EMC/2012
<220>
   <223> protein 3
<400> 11
   Met Arg Val Gln Arg Pro Pro Thr Leu Leu Leu Val Phe Ser Leu Ser
   1               5                   10                  15
   Leu Leu Val Thr Ala Ser Ser Lys Pro Leu Tyr Val Pro Glu His Cys
               20                  25                  30
   Gln Asn Tyr Ser Gly Cys Met Leu Arg Ala Cys Ile Lys Thr Ala Gln
           35                  40                  45
   Ala Asp Thr Ala Gly Leu Tyr Thr Asn Phe Arg Ile Asp Val Pro Ser
       50                  55                  60
   Ala Glu Ser Thr Gly Thr Gln Ser Val Ser Val Asp Leu Glu Ser Thr
   65                  70                  75                  80
   Ser Thr His Asp Gly Pro Thr Glu His Val Thr Ser Val Asn Leu Phe
                   85                  90                  95
   Asp Val Gly Tyr Ser Val Asn
               100
<210> 12
   <211> 109
   <212> PRT
   <213> Human betacoronavirus 2c EMC/2012
<220>
   <223> protein 4a
<400> 12
   Met Asp Tyr Val Ser Leu Leu Asn Gln Ile Trp Gln Lys Tyr Leu Asn
   1               5                   10                  15
   Ser Pro Tyr Thr Thr Cys Leu Tyr Ile Pro Lys Pro Thr Ala Lys Tyr
               20                  25                  30
   Thr Pro Leu Val Gly Thr Ser Leu His Pro Val Leu Trp Asn Cys Gln
           35                  40                  45
   Leu Ser Phe Ala Gly Tyr Thr Glu Ser Ala Val Asn Ser Thr Lys Ala
       50                  55                  60
   Leu Ala Lys Gln Asp Ala Ala Gln Arg Ile Ala Trp Leu Leu His Lys
   65                  70                  75                  80
   Asp Gly Gly Ile Pro Asp Gly Cys Ser Leu Tyr Leu Arg His Ser Ser
                   85                  90                  95
   Leu Phe Ala Gln Ser Glu Glu Glu Glu Pro Phe Ser Asn
               100                 105
<210> 13
   <211> 246
   <212> PRT
   <213> Human betacoronavirus 2c EMC/2012
<220>
   <223> protein 4b
<400> 13
   Met Glu Glu Ser Leu Met Asp Val Pro Ser Thr Ser Gly Thr Gln Val
   1               5                   10                  15
   Tyr Ser Arg Lys Ala Arg Lys Arg Ser His Ser Pro Thr Lys Lys Leu
               20                  25                  30
   Arg Tyr Val Lys Arg Arg Phe Ser Leu Leu Arg His Glu Asp Leu Ser
           35                  40                  45
   Val Ile Val Gln Pro Thr His Tyr Val Arg Val Thr Phe Ser Asp Pro
       50                  55                  60
   Asn Met Trp Tyr Leu Arg Ser Gly His His Leu His Ser Val His Asn
   65                  70                  75                  80
   Trp Leu Lys Pro Tyr Gly Gly Gln Pro Val Ser Glu Tyr His Ile Thr
                   85                  90                  95
   Leu Ala Leu Leu Asn Leu Thr Asp Glu Asp Leu Ala Arg Asp Phe Ser
               100                 105                 110
   Pro Ile Ala Leu Phe Leu Arg Asn Val Arg Phe Glu Leu His Glu Phe
           115                 120                 125
   Ala Leu Leu Arg Lys Thr Leu Val Leu Asn Ala Ser Glu Ile Tyr Cys
       130                 135                 140
   Ala Asn Ile His Arg Phe Lys Pro Val Tyr Arg Val Asn Thr Ala Ile
   145                 150                 155                 160
   Pro Thr Ile Lys Asp Trp Leu Leu Val Gln Gly Phe Ser Leu Tyr His
                   165                 170                 175
   Ser Gly Leu Pro Leu His Met Ser Ile Ser Lys Leu His Ala Leu Asp
               180                 185                 190
   Asp Val Thr Arg Asn Tyr Ile Ile Thr Met Pro Cys Phe Arg Thr Tyr
           195                 200                 205
   Pro Gln Gln Met Phe Val Thr Pro Leu Ala Val Asp Val Val Ser Ile
       210                 215                 220
   Arg Ser Ser Asn Gln Gly Asn Lys Gln Ile Val His Ser Tyr Pro Ile
   225                 230                 235                 240
   Leu His His Pro Gly Phe
                   245
<210> 14
   <211> 224
   <212> PRT
   <213> Human betacoronavirus 2c EMC/2012
<220>
   <223> protein 5
<400> 14
   Met Ala Phe Ser Ala Ser Leu Phe Lys Pro Val Gln Leu Val Pro Val
   1               5                   10                  15
   Ser Pro Ala Phe His Arg Ile Glu Ser Thr Asp Ser Ile Val Phe Thr
               20                  25                  30
   Tyr Ile Pro Ala Ser Gly Tyr Val Ala Ala Leu Ala Val Asn Val Cys
           35                  40                  45
   Leu Ile Pro Leu Leu Leu Leu Leu Arg Gln Asp Thr Cys Arg Arg Ser
       50                  55                  60
   Ile Ile Arg Thr Met Val Leu Tyr Phe Leu Val Leu Tyr Asn Phe Leu
   65                  70                  75                  80
   Leu Ala Ile Val Leu Val Asn Gly Val His Tyr Pro Thr Gly Ser Cys
                   85                  90                  95
   Leu Ile Ala Phe Leu Val Ile Leu Ile Ile Leu Trp Phe Val Asp Arg
               100                 105                 110
   Ile Arg Phe Cys Leu Met Leu Asn Ser Tyr Ile Pro Leu Phe Asp Met
           115                 120                 125
   Arg Ser His Phe Ile Arg Val Ser Thr Val Ser Ser His Gly Met Val
       130                 135                 140
   Pro Val Ile His Thr Lys Pro Leu Phe Ile Arg Asn Phe Asp Gln Arg
   145                 150                 155                 160
   Cys Ser Cys Ser Arg Cys Phe Tyr Leu His Ser Ser Thr Tyr Ile Glu
                   165                 170                 175
   Cys Thr Tyr Ile Ser Arg Phe Ser Lys Ile Ser Leu Val Ser Val Thr
               180                 185                 190
   Asp Phe Ser Leu Asn Gly Asn Val Ser Thr Val Phe Val Pro Ala Thr
           195                 200                 205
   Arg Asp Ser Val Pro Leu His Ile Ile Ala Pro Ser Ser Leu Ile Val
       210                 215                 220